# EUROPÄISCHE PATENTSCHRIFT

(11) **EP 1 068 299 B1**
(45) Veröffentlichungstag und Bekanntmachung des Hinweises auf die Patenterteilung: **06.12.2006**
(21) Anmeldenummer: 99914560.0
(22) Anmeldetag: 31.03.1999
(51) Int. Cl.: C12N 5/08, A61K 35/14

(54) **VERWENDUNG VON STIMULIERTEN MONONUKLEÄREN ZELLEN DES PERIPHEREN BLUTES ZUR BEHANDLUNG VON MIT DEM GEHIRN ASSOZIIERTEN ERKRANKUNGEN, STÖRUNGEN UND SCHÄDIGUNGEN**
USE OF STIMULATED PERIPHERAL BLOOD MONONUCLEAR CELLS FOR THE TREATMENT OF BRAIN-RELATED DISEASES, DISORDERS AND DAMAGE
UTILISATION DE CELLULES MONONUCLEAIRES STIMULEES DU SANG PERIPHERIQUE POUR LE TRAITEMENT D'AFFECTIONS, DE PERTURBATIONS ET DE LESIONS ASSOCIEES AU CERVEAU

(30) Priorität: 01.04.1998 DE 19814701
(43) Veröffentlichungstag der Anmeldung: 17.01.2001
(73) Patentinhaber: Wank, Rudolf, 80469 München (DE)
(72) Erfinder: Wank, Rudolf, 80469 München (DE)
(74) Vertreter: Wibbelmann, Jobst
(86) Internationale Anmeldenummer: PCT/EP1999/002225
(87) Internationale Veröffentlichungsnummer: WO 1999/050393

(56) Entgegenhaltungen:
- EP-A- 0 346 022
- WO-A-91/03250
- WO-A-95/20649
- SIROTA P ET AL: "Increased interleukin-1 and interleukin-3 like activity in schizophrenic patients." PROGRESS IN NEURO-PSYCHOPHARMACOLOGY AND BIOLOGICAL PSYCHIATRY, (1995 JAN) 19 (1) 75-83. , XP002115877

## Beschreibung

Die Erfindung betrifft die Verwendung von stimulierten oder aktivierten mononukleären Zellen des peripheren Blutes zur Behandlung von verschiedenen mit dem Gehirn assoziierten Erkrankungen, Störungen und Schädigungen, insbesondere psychiatrischen Erkrankungen, wie manisch-depressiver Krankheit oder Schizophrenie, depressiven Syndromen ohne endogene Ursachen, Gehirnentwicklungsstörungen in und nach der Embryonalzeit, z.B. beim Down-Syndrom, Autismus, unfall- oder aufgrund anderer Ursachen geschädigten Gehirnen und Parkinson-Syndrom. Im Rahmen dieser Anmeldung werden die Begriffe "Stimulierung" und "Aktivierung" stets synonym gebraucht.

Geisteskrankheiten machen gemäß dem WHO-Report von 1996 etwa 11% aller Krankheiten aus. Bei den bei Geisteskrankheiten auftretenden Symptomen fehlen pathogene Faktoren, die diese Symptome erklären könnten.

Die letzte Erkrankung, die aus der Gruppe der Geisteskrankheiten ausgegliedert wurde, da ein dafür verantwortlicher pathogener Faktor identifiziert werden konnte, war die Demenz, die durch die Spirochaeta treponema pallida verursacht wird. Bei dem nur bei ca. 30% der unbehandelten Personen mit latenter Infektion auftretenden dritten Stadium dieser Syphiliserkrankung treten Symptome einer Demenz auf. Da Penicillin eine effektive Behandlung sämtlicher Stadien der Syphilis ermöglicht, beschäftigten sich nur wenige Untersuchungen mit der Frage, ob eine immungenetische Veranlagung, eine Variante dieser Spirochaeta oder andere Sekundärfaktoren zu diesem dritten Syphilisstadium führen.

Eine ähnliche Beteiligung einer immungenetischen Veranlagung oder eines pathogenen Faktors kann bei HIV-Infektionen festgestellt werden, die nur bei einem Bruchteil der Patienten paranoide Symptome hervorrufen.

Zwillings-, Familien- und Adoptionsstudien zeigten ein erhöhtes Risiko für psychiatrische Erkrankungen mit dem Anteil gemeinsamer Erbanlagen. Die genetische Analyse wird höchstwahrscheinlich durch klinische Heterogenität, die Beteiligung mehrerer Genorte an der Erzeugung von Phänotypen, unvollständige Penetranz, den Einfluß des Geschlechts und alternative Chromosomen erschwert.

Die Probleme von Kopplungsstudien bei schizophrenen Erkrankungen und manisch-depressiver Krankheit wurden unlängst in Übersichtsartikeln dargestellt (Peltonen, L., Nature 1995: 378, 665-666; MacKinnon, D.F., et al., Annu. Rev. Neurosci. 1997:20, 335-73). Bei Schizophrenie wurden genetische Faktoren für Vulnerabilität von mehreren Gruppen auf dem kurzen Arm des Chromosoms 6 lokalisiert (Straub, R.E., et al., Nature Genet. 1995: 11, 287-93; Moises, H.W., et al., Nature Genet. 1995: 11, 321-324; Schwab, S.G., et al., Nature Genet. 1995: 11, 325-327; Antonarakis, S.E., et al., Nature Genet. 1995: 11, 235-236). Auf dem Chromosom 6 sind die Gene des HLA-Komplexes lokalisiert. Der hochgradige Polymorphismus des HLA-Systems bringt eine variable Immunreaktivität bei jedem Individuum mit sich. Manche Varianten eines Genortes können häufig mit Varianten eines anderen Genortes gekoppelt gefunden werden (Kopplungsungleichgewicht). Solche Kopplungsungleichgewichte zwischen Genvarianten können in einzelnen Populationen sehr verschieden sein. Dies kann sowohl regionale Persistenz von pathogenen Faktoren als auch unterschiedliche Assoziierung der Krankheiten mit HLA-Faktoren und pathogenen Faktoren erklären. Immungenetische Studien, die serologische Verfahren einsetzen, haben herausgefunden, daß mehrere HLA-Klasse I- und -Klasse II-Antigene mit manisch-depressiver Krankheit und schizophrenen Erkrankungen assoziiert sind (siehe z.B. Ivanyi, D., et al., Tissue Antigens 1976: 8, 217-220; Smeraldi, E., et al., Biol. Psychiatry 1976: 11, 665 ff.). Unlängst hat eine Forschergruppe eine signifikant erhöhte Häufigkeit der mit Narkolepsie assoziierten Antigene HLA-DR15 und -DQ6 bei chronisch an Schizophrenie erkrankten Patienten festgestellt (Douglass, A.B., et al., Biol. Psychiatry 1993: 34, 773-780). Zusätzlich wurde unlängst bei schizophrenen Patienten eine signifikante Vermehrung der HLA-Allele DRB1*1501 und DQB1*0602 gefunden, die diese Moleküle kodieren. Dementsprechend ist dasselbe HLA-Allel DQB1*0602 mit Narkolepsie, multipler Sklerose und Schizophrenie assoziiert. (Großkopf, A., Müller, N., Malo, A., Wank, R.: Potential role for the narcolepsy and multiple sclerosis associated HLA allele DQB1*0602 in schizophrenia subtypes; Schiz. Res. 1998, im Druck).

Es ist heute durch eine Vielzahl von Veröffentlichungen belegt, daß die Zellen des Immunsystems die meisten Zytokine des Zentralnervensystems produzieren und daß Zellen des Nervensystems viele Zytokine des Immunsystems produzieren (Blalok, J.E., The Immunologist 1994: 2, 8-15; Wilder, R.L., Annu. Rev. Immunol. 1995: 13, 307-338).

Über eine bemerkenswerte Beobachtung der komplizierten bidirektionalen Wechselwirkungen zwischen dem Zentralnervensystem und dem Immunsystem wurde hinsichtlich des Immunzytokins Interleukin 2 (IL-2) berichtet, das Schizophrenie-Symptome bei Tumorpatienten hervorrief. Die Symptome verschwanden, nachdem die IL-2-Behandlung unterbrochen worden war (Denicoff, K.D., et al., Ann. Intern. Med. 1987: 107, 293-300). Diese Beobachtung korreliert mit dem Bericht über erhöhte IL-2-Konzentrationen in der Cerebrospinalflüssigkeit von schizophrenen Patienten (Licino, J., et al., Am. J. Psychiatry 1993: 150:9, 1408-1410). Erhöhte IL-2-Konzentrationen in der Cerebrospinalflüssigkeit nach einer Behandlung mit Neuroleptika sind die genauesten Indikatoren für Rückfälle bei Schizophreniepatienten (McAllister, C.G., et al., Am. J. Psychiatry 1995: 152:9, 1291-1297).

Pathognostische Zytokinkonzentrationen, wie sie für die IL-2-Konzentrationen in der Cerebrospinalflüssigkeit von schizophrenen Patienten berichtet werden, könnten durch ein Versagen in zwei Systemen, dem Zentralnervensystem oder dem Immunsystem, oder in -beiden hervorgerufen werden.

Bei vielen anderen Erkrankungen und insbesondere bei Infektionskrankheiten kann eine Beeinträchtigung des Kommunikationssystems beobachtet werden. Hinsichtlich des chronischen Müdigkeitssyndroms ("chronical fatigue syndrome") wurde eine mögliche Induktion auch durch Epstein-Barr-Virus postuliert (Holmes, G.P., et al., JAMA 1987: 257, 2297-2302). Darüberhinaus ist jedoch bislang nur geringe Aufmerksamkeit auf die Langzeitkonsequenzen von geringgradigen, persistierenden Infektionen als Ursache von mit dem Gehirn assoziierten Erkrankungen und insbesondere Geisteskrankheiten, gerichtet worden. Aus der EP 0 346 022 A ist ein in vitro Verfahren bekannt, in dem zytotoxische T-Lymphozyten aufbereitet werden, um in vivo HIV-infizierte Zellen zu zerstören.

Es besteht daher ein Bedarf für ein Mittel zur Behandlung von mit dem Gehirn assoziierten Erkrankungen, Störungen und Schädigungen, die das mögliche Vorhandensein von ggf. über lange Zeit bestehenden geringgradigen persistierenden Infektionen als (Mit)Ursache von Geisteskrankheiten oder allgemein von mit dem Gehirn assoziierten Erkrankungen, Störungen und Schädigungen mitberücksichtigt. Zu berücksichtigen sind in diesem Zusammenhang insbesondere Faktoren, die die Funktion von Lymphozytensubpopulationen beeinträchtigen und zur Verschlechterung des mentalen Wohlbefindens beigetragen haben könnten.

Die WO 91 03250 A beschreibt die Verwendung von stimulierten Leukozyten zur Behandlung von mit dem Gehirn assoziierten Erkrankungen, Störungen und Schädigungen, bzw. zur Behandlung des Parkinson-Syndroms. Dem gegenüber schlägt die Erfindung vor, dass stimulierte mononukleäre Zellen des peripheren Blutes mittels einer Injektion eingesetzt werden. Gegenstand der Erfindung ist somit die Verwendung von stimulierten mononukleären Zellen des peripheren Bluts (PBMC) zur Bereitstellung eines Mittels zur Behandlung von mit dem Gehirn assoziierten Erkrankungen, Störungen und Schädigungen, wobei die stimulierte PBMC mittels einer intradermalen oder intramuskulären Injektion beispielweise in einer Dosismenge von 1 - 30 x 10⁶ Zellen pro Injektion eingesetzt werden.

Erfindungsgemäß können stimulierte mononukleäre Zellen des peripheren Blutes (PBMC) zur Behandlung von verschiedenen mit dem Gehirn assoziierten Erkrankungen, Störungen und Schädigungen, insbesondere psychiatrischen Erkrankungen, wie manisch-depressiver Krankheit oder Schizophrenie, depressiven Syndromen ohne endogene Ursachen, Gehirnentwicklungsstörungen in und nach der Embryonalzeit, z.B. beim Down-Syndrom, Autismus und unfall-oder aufgrund anderer Ursachen geschädigten Gehirnen oder Parkinson-Syndrom, eingesetzt werden. Wie sich bereits aus der vorstehenden Auflistung exemplarischer mit dem Gehirn assoziierter Erkrankungen, Störungen und Schädigungen ergibt, werden von diesem Begriff keine Krebserkrankungen umfaßt.

Gemäß der Erfindung werden in Zusammenhang mit den genannten Erkrankungen, Störungen und Schädigungen die stimulierten mononukleären Zellen des peripheren Blutes (PBMC) als primäres Therapeutikum wirksam, benötigen also nicht den Zusatz weiterer therapeutisch wirksamer Agenzien. Insbesondere ist für die erfindungsgemäß erzielbaren Wirkungen bei den genannten Erkrankungen, Störungen oder Schädigungen kein zusätzlicher Einsatz transplantierter allogener Zellen, wie z.B. fötaler Neurozyten, die gegenwärtig im Rahmen von sogenannten
"Lebendzelltransplantat-Therapien" ("vital cell transplant therapy") eingesetzt werden, erforderlich und wird grundsätzlich auch nicht beabsichtigt.

Die Stimulierung oder Aktivierung der mononukleären Zellen (PBMC) kann "einstufig", über eine "vorgeschaltete" Aktivierung der T-Lymphozyten in den PBMC, die dann in stimuliertem Zustand die übrigen Zellen stimulieren, oder in einer ganz speziellen Ausführungsform über sogenannte "Kaskaden-Gedächtnisprägung" ("cascade priming"), die nachfolgend detaillierter erläutert wird, erfolgen.

Bei der "einstufigen Vorgehensweise" kann die Gesamtheit oder zumindest ein Großteil der PBMC mit inaktivierten Keimen oder Mikroorganismen primär stimuliert werden, wobei durch Sekundärereignisse nach der Primärstimulierung, insbesondere auch durch eine Aktivierung der T-Lymphozyten in der PBMC-Population, die Stimulierung noch verstärkt werden kann.

Die Inaktivierung der Keime oder Mikroorganismen kann dabei beispielsweise durch Bestrahlen mit energiereicher Strahlung, in der Regel mit γ-Strahlen, erfolgen. Alternativ können für die Aktivierung der PBMC auch Proteine dieser Keime oder Mikroorganismen eingesetzt werden, und zwar Gesamtproteine wie auch Peptide von diesen. Beispielsweise können hierfür sogenannte "Superantigene" von Staphylococcus aureus oder Proteine von Tuberkulosebakterien, wie sie auch bei der Krebstherapie eingesetzt werden (BCG-Impfung), eingesetzt werden. Auch Proteine von Herpes simplex- oder Epstein-Barr-Virus sind hierfür geeignet.

Im Falle einer "vorgeschalteten" Stimulierung oder Aktivierung im wesentlichen nur der T-Lymphozyten in der PBMC-Präparation erfolgt die Aktivierung der übrigen PBMC mittels der stimulierten T-Lymphozyten durch direkten Kontakt der T-Lymphozyten mit den PBMC über eine Bindung an die T-Zellrezeptoren und/oder die Stimulierung/Aktivierung wird durch Zytokine, die durch die aktivierten T-Lymphozyten produziert und freigesetzt werden und ihrerseits mit den übrigen PBMC über spezifische Rezeptoren der letzteren in Kontakt treten, vermittelt. Im diesem Falle könnte man somit von einer "zweistufigen" Aktivierung sprechen. Bei "einstufiger" wie bei "zweistufiger" Aktivierung werden wie bei einer Impfung Lymphozyten zu T- und B-Gedächtniszellen ausgebildet. Bei einer konventionellen Impfung wird ein bekannter "Problemkeim" aufbereitet, bei der Aktivierung von PBMC werden ein oder mehrere, bekannte oder unbekannte, "Problemkeime" den mit dem Betriebsstoff IL-2 versorgten T-Lymphozyten präsentiert.

Die erforderliche Stimulierung oder Aktivierung der T-Lymphozyten kann durch eine Vielzahl von Maßnahmen erreicht werden, die dem Fachmann wohlbekannt sind.

Die Aktivierung erfolgt dabei in der Regel über die Einwirkung chemischer oder biologischer Stoffe von außen auf die T-Lymphozyten, die meist über die Bindung dieser Stoffe an auf den T-Lymphozyten befindliche hochspezifische Rezeptoren, Corezeptoren oder Signalmoleküle vermittelt wird.

Die Aktivierung von T-Lymphozyten vermittelnde Rezeptoren, Corezeptoren und Signalmoleküle werden z.T. spezifisch für den jeweiligen Typ von T-Lymphozyt und/oder z.T. auch spezifisch für die Entwicklungsstufe eines T-Lymphozyten durch diese exprimiert. Diese Rezeptoren und Signalmoleküle sind Membranproteine, die aufgrunddessen oder im Zusammenwirken mit ihnen assoziierten Transmembranproteinen "Informationen" aus dem Raum außerhalb der Zelle in das Innere der Zelle weiterleiten können.

Von T-Lymphozyten exprimierte Rezeptoren, Corezeptoren und Signalmoleküle, die eine Aktivierung derselben vermitteln können, sind beispielsweise der αβ-T-Zellrezeptor, die CD3-Protein-gruppe, das CD2- und das CD4-Protein wie auch das CD28-Protein der T-Helferzellen, einer besonderen Klasse von T-Lymphozyten. Möglicherweise spielt in diesem Zusammenhang auch das CD8-Protein eine Rolle.

T-Lymphozyten können außer durch die Moleküle, die unter physiologischen oder Zellkulturbedingungen normalerweise spezifisch an einen jeweiligen Rezeptor, Corezeptor oder ein jeweiliges Signalmolekül binden, um eine Zellreaktion der T-Lymphozyten hervorzurufen, auch mittels Antikörpern stimuliert werden, die spezifisch an diese Rezeptoren, Corezeptoren bzw. Signalmoleküle binden. Derartige Antikörper können beispielsweise gegen den extrazellulären Abschnitt des αβ-T-Zellrezeptors oder gegen den extrazellulären Abschnitt eines CD3-Proteins auf den T-Lymphozyten gerichtet sein.

Für eine Aktivierung von T-Lymphozyten können derartige Antikörper beispielsweise immobilisiert an einen festen Träger, z.B. an die Oberfläche von Mikrotiterplatten oder auch von Körnchen aus Polymermaterial, für eine leichtere Abtrennung nach der Einwirkung eingesetzt werden. Die Antikörper können monoklonal oder polyklonal sein. Verfahren zur Erzeugung von für den genannten Zweck geeigneten monoklonalen und polyklonalen Antikörper sowie Verfahren zur Immobilisierung derselben sind dem Fachmann wohlbekannt. Darüberhinaus sind derartige Antikörper z.T. auch kommerziell erhältlich.

Dabei kann ggf. das Problem auftreten, daß die unphysiologische Stimulierung von T-Lymphozyten allein mittels eines Antikörpers zum Zelltod führt, also Apoptosevorgänge in den stimulierten Zellen auslöst. Dieses Problem kann durch Zusätze geeigneter Zytokine, wie Interleukine, umgangen werden. Derartige Maßnahmen sind dem Fachmann wohlbekannt.

Z.B. führt normalerweise die alleinige Stimulierung mit einem Antikörper, der an ein CD3-Protein bindet, zum Zelltod, sofern man die Zellen nicht mit Interleukin 2 vor dem programmierten Zelltod oder Apoptose rettet. Ein Zusatz von Interleukin 2 zur Zellkultur bewirkt folglich eine Expansion der kultivierten Zellen und ermöglicht, daß aufgrund des Vorhandenseins weiterer PBMC-Zellarten ein Kontakt zwischen den stimulierten T-Lymphozyten und diesen weiteren Zellen der PBMC zur Stimulierung der letzteren erfolgen kann.

Die tiefgreifendste Stimulierung der T-Zelle erfolgt über den αβ-T-Zellrezeptor. Der T-Zellrezeptor wird von verschiedenen CD3-Proteinmolekülen flankiert, und in vivo wird in der Regel der Gesamtkomplex aus αβ-T-Zellrezeptor und CD3-Molekülketten stimuliert. Die CD3-Proteine dienen dabei der Signalweiterleitung in das Zellinnere. Meist werden gleichzeitig Aktivierungssignale über das CD4- und/oder das CD8-Protein in das Zellinnere weitergeleitet.

Beispielsweise erfolgt die Stimulierung von T-Lymphozyten durch allogene Zellen oder Proteine von diesen über den αβ-T-Zellrezeptor.

Der αβ-T-Zellrezeptor ist hochspezialisiert. Er erkennt im Blut gelöste Proteine normalerweise nicht, sofern sie nicht von anderen mononukleären Zellen, vor allem von B-Lymphozyten, Monozyten und dendritischen Zellen, den sogenannten Antigen-präsentierenden Zellen (APC), präsentiert werden. Die Präsentation solcher Proteine durch die APC erfolgt in Form von kurzkettigen Peptiden, die stets in vorzugsweise autologe, d.h. körpereigene HLA-Moleküle eingebunden sein müssen. Die Peptide werden also über die HLA-Moleküle oder in einem bestimmten HLA- oder MHC-Kontext präsentiert. Es können alternativ auch allogene APC mit dementsprechend identischen HLA-Molekülen eingesetzt werden.

Das CD28-Protein der T-Helferzellen aus der Klasse der T-Lymphozyten vermittelt als Signalmolekül eine Zellaktivierung unter physiologischen Bedingungen durch Bindung z.B. des B7-oder synonym B7-1-Moleküls, das konstitutiv auf dendritischen Zellen exprimiert wird und auf B-Zellen und Monozyten/Makrophagen induziert werden kann, oder durch Bindung des B7-2-Moleküls, das auf APC exprimiert wird, als natürliche Liganden.

T-Lymphozyten können ferner durch Zytokine, die z.B. von mononukleären Zellen (PBMC) oder Gehirnzellen produziert werden, oder alternativ mit autokrin produzierten Zytokinen, z.B. Interleukin 2, stimuliert oder aktiviert werden. Die Stimulierung wird auch in diesem Falle in der Regel über spezifische Rezeptoren, die die Zytokine binden, auf den T-Lymphozyten vermittelt.

T-Lymphozyten können auch durch Lektine, z.B. Phytohaemagglutinin (PHA) oder Concanavalin A, stimuliert werden. Alternativ kann eine Stimulierung der T-Lymphozyten mittels chemischer Substanzen, wie Calcium-Ionophoren und chemischen Analoga davon, oder durch allogene, natürliche oder gentechnisch manipulierte und xenogene, vor allem gentechnisch manipulierte Tierzellen erzielt werden. Im Beisein anderer mononukleärer Zellen kann dies zu einem sehr ähnlichen Effekt wie bei einer Zellstimulierung mittels eines Antikörpers über das CD3-Protein führen.

Es ist vorgesehen, daß die Stimulierung der PBMC und/oder der T-Lymphozyten für die erfindundgsgemäße Verwendung auch durch mehrere der vorstehend genannten Maßnahmen gleichzeitig oder nacheinander erfolgen kann.

Es können auch nach Terminierung der Zellaktivierung und Expansion PBMC Populationen oder T-Zellsubpopulationen entfernt werden. In der Regel wird jedoch für die Erzeugung der stimulierten oder aktivierten PBMC durch das "zweistufige Verfahren" mit vorgeschalteter Stimulierung von im wesentlichen nur des T-Lymphozytenanteils eine PBMC-Zellmischung eingesetzt, die neben den zu stimulierenden T-Lymphozyten auch andere mononukleäre Zellen des peripheren Bluts (PBMC), z.B. B-Lymphozyten, Monozyten/Makrophagen, dendritische Zellen und NK-Zellen, enthält, so daß beide "Stufen" der Stimulierung in ein und derselben Zellkultur stattfinden.

Es wird gleichfalls in Betracht gezogen, eine autologe oder allogene T-Lymphozytenpopulation zunächst isoliert, wie vorstehend erläutert, zu stimulieren und erst dann PBMC für eine Stimulierung der letzteren zuzusetzen. Alternativ können naive PBMC "einstufig" oder "zweistufig" stimulierten PBMC zugesetzt werden, wodurch diese naiven PBMC ihrerseits in einer weiteren Stufe stimuliert werden. Diese Varianten werden unter dem Begriff "Kaskaden-Gedächtnisprägung" oder "cascade priming" zusammengefaßt und die daraus resultierenden Zellen als Kaskadengedächtnisgeprägte Zellen oder "cascade primed" Zellen bezeichnet.

Für die primäre, ein- oder zweistufige Stimulierung der T-Lymphozyten oder der PBMC können sämtliche vorstehend erläuterten Stimulierungsmöglichkeiten eingesetzt werden. Beispielsweise kann die Stimulierung der T-Lymphozyten allein oder im PBMC-Verband mittels CD3-Antikörpern unter Zusatz von IL-2 erfolgen.

Nach einer Inkubationszeit für die Primärstimulierung, die üblicherweise geringer ist als jene für eine ein- oder zweistufige Aktivierung, z.B. nur 3-10 h, beispielsweise 3-6 h, gegenüber 2 - 20 h für die ein- oder zweistufige Aktivierung werden dann den stimulierten T-Lymphozyten bzw. PBMC naive, also nicht-stimulierte PBMC zugesetzt. Nach einer weitere Inkubationsdauer von 24 h bis maximal 10 Tagen, beispielsweise zwischen 4 und 6 Tagen ist die Stimulierung auch der naiven PBMC erfolgt, so daß die Zellpräparation erfindungsgemäß eingesetzt werden kann. Während dieser weiteren Inkubation erfolgt somit eine kontinuierliche Weiterstimulierung der bereits primär stimulierten Zellen, während die neu hinzugefügten naiven PBMC hauptsächlich über die in dem ersten Schritt primär stimulierten Zellen stimuliert werden. Das zahlenmäßige Verhältnis der primär stimulierten Zellen zu den naiven PBMC beträgt dabei in der Regel zwischen 1:1 und 1:10, kann aber in Einzelfällen auch darunter oder darüber liegen. üblicherweise wird ein Verhältnis von ungefähr 1:1 eingesetzt.

Bei dieser Stimulierungsvariante wird davon ausgegangen, daß bei Verwendung von PBMC für die primäre Stimulation die Stimulierung der für den weiteren Inkubationsschritt zugesetzten naiven Zellen ganz überwiegend über die aktivierten APC in der primär stimulierten Zellpopulation erfolgt, d.h. hochspezifisch über den T-Zell-Rezeptor der T-Lymphozyten (CD4-Helfer- und CD8-Killerzellen) in der naiven PBMC-Population.

Wie nachfolgend näher erläutert werden wird, weisen die auf diese Weise durch "cascade priming" hergestellten Zellen eine deutlich gesteigerte Effektivität bei der erfindungsgemäßen Verwendung zur Behandlung von mit dem Gehirn assoziierten Erkrankungen, Störungen und Schädigungen auf.

Dem Fachmann sind diverse Verfahren zur Stimulierung von PBMC und/oder T-Lymphozyten mittels der genannten Maßnahmen geläufig.

Die für die Stimulierung eingesetzten PBMC stammen in der Regel von dem zu behandelnden Patienten, dem sie nach erfolgter Stimulierung und ggf. darauffolgender Expansion erneut verabreicht werden.

Ohne daß beabsichtigt ist, auf eine bestimmte Theorie zur Erklärung der feststellbaren Wirkungen derart aktivierter mononukleärer Zellen festgelegt zu werden, werden zwei Wirkungsmechanismen hierfür in Betracht gezogen.
1) Der erste in Betracht gezogene Wirkungsmechanismus beruht auf der Hypothese, daß Lymphozyten, die aufgrund der von ihnen produzierten Zytokine auch mit dem ZNS kommunizieren, jedoch durch unerkannte oder unterschätzte chronische Infektionen in ihrer Funktion oder Aktivität u.a. hinsichtlich der Zytokinproduktion beeinträchtigt sein könnten, über die veränderte Zytokinproduktion Zellen des ZNS abträglich beeinflussen könnten, die für ihren Normalzustand auch von der normalen Zytokinproduktion bzw. der normalen Produktion bestimmter einzelner Zytokine durch diese Immunzellen abhängen. So wurden z.B. abnormale physiologische Konzentrationen des Zytokins Interleukin 2 wiederholt mit der Manifestierung psychiatrischer Krankheiten in Verbindung gebracht.
   In diesem Zusammenhang könnten die durch die aktivierten T-Lymphozyten produzierten und ausgeschiedenen Zytokine die als Antigen-präsentierende Zellen (APC) bezeichneten Zelltypen der PBMC, nämlich B-Lymphozyten, Monozyten und dendritische Zellen, befähigen, bakterielle und virale Proteine, die sich in diesen befinden, auf ihrer Oberfläche für T-Lymphozyten zu präsentieren und damit einer Immunreaktion zugänglich zu machen. Dies betrifft insbesondere bakterielle und virale Proteine, die Produkte sogenannter "verschwiegener", insbesondere chronischer Infekte sind und normalerweise sich einer Präsentation als Antigene auf den APC entziehen. Von zwei Viren, dem Epstein-Barr-Virus und dem Herpes simplex-Virus ist bekannt, daß sie infizierte Zellen daran hindern können, virale Peptide zu präsentieren. Die genannten Viren ermöglichen oder unterstützen demnach die Ausbildung derartiger "verschwiegener" Infekte.
   Als Beispiel für derartige "verschwiegene", insbesondere chronische Infektionen mit möglicher Auswirkung auf bestimmte der vorstehend genannten, mit dem Gehirn assoziierten Erkrankungen kann das Epstein-Barr-Virus mit möglichen Auswirkungen auf Schizophrenie genannt werden. Auch für das Hepatitis C-Virus wird eine Rolle zumindest als Kofaktor bei Schizophrenie vermutet.
   Die aktivierten T-Lymphozyten stimulieren über die von ihnen ausgeschiedenen Zytokine die APC innerhalb der PBMC des jeweiligen Patienten, die von Pathogenen stammenden und normalerweise nicht präsentierten "Problemproteine" zu prozessieren, als Peptide an der Zelloberfläche zu präsentieren und einer Immunreaktion zugänglich zu machen. In Zellkultur konnte nachgewiesen werden, daß dies zu einer Eliminierung infizierter Zellen in der Zellkultur führt. Es wird vermutet, daß APC sowohl in der Zellkultur als auch in der Haut durch die Zellinjektion - und hier vor allem dendritische Zellen - dazu stimuliert werden, "alles" an zurückgehaltenen Peptiden zu präsentieren.
   Durch die erfindungsgemäße Verwendung der stimulierten PBMC kann auf diese Weise eine Verringerung der Belastung durch pathogene Faktoren erreicht werden, die insbesondere über die Beeinträchtigung des Immunsystems möglicherweise einen Einfluß auf die zu behandelnde Krankheit ausüben.
   Einen besonderen Vorteil stellt es dabei dar, daß es nicht erforderlich ist, daß die pathogenen Faktoren bekannt sind. Ein weiterer Vorteil ist, daß eine vorab erfolgende Aktivierung von T-Lymphozyten beispielsweise über CD3 die Spezifität der T-Lymphozyten nicht präjudiziert.
2) Der zweite mögliche Wirkungsmechanismus beruht auf der Hypothese, daß durch die PBMC, also Zellen des Immunsystems, Stoffe produziert und für das ZNS bereitgestellt werden könnten, die für dieses notwendig sind, jedoch aufgrund der Erkrankung, Störung oder Schädigung durch die Zellen des ZNS nicht oder nicht in ausreichender Menge hergestellt werden können.

Gemäß dieser Hypothese könnten die aktivierten PBMC schützende, ggf. im Gehirn nicht ausreichend vorliegende Stoffe produzieren, die dort zur Linderung der mit der jeweiligen Erkrankung verbundenen Symptome wirksam werden. Als Beispiele können hier Propiomelanocortin, BDNF ("brain-derived neurotrophic factor") und diverse Neurotrophine, z.B. Neurotrophin-3 (NT3) genannt werden. Wie nachgewiesen werden konnte, werden sowohl BDNF als auch NT3 von Immunzellen aufgenommen, aber auch produziert.

Die beiden letztgenannten Faktoren BDNF und NT3 werden derzeit als wichtigste Schutz- und Entwicklungsfaktoren für Neuronen anerkannt. Dieser Effekt könnte insbesondere für die Wirksamkeit stimulierter PBMC bei der Behandlung von Gehirnentwicklungsstörungen in und nach der Embryonalzeit, wie z.B. beim Down-Syndrom, von Autismus, der von vielen Fachleuten als Entwicklungsstörung angesehen wird, und von Patienten mit unfall-oder aufgrund anderer Ursachen geschädigten Gehirnen, z.B. von Patienten im chronischen Koma, von großer Bedeutung sein.

Nach einer Stimulierung produzieren neben den T-Lymphozyten auch alle anderen Zellarten der PBMC verschiedene Zytokine gemäß einem für jeden Zelltyp besonderen Muster. So produzieren stimulierte T-Lymphozyten je nach Gruppenzugehörigkeit z.B. Interleukin 2, Interleukin 12 und Interferon γ, TNFβ, aber auch Interleukin 3, 4, 5, 6, 10, TGFβ und GM-CSF. TNFβ wird vor allem von T-Lymphozyten des Helfer-1-Typs exprimiert, TGFβ insbesondere von T-Lymphozyten vom Helfer-2-Typ. Stimulierte B-Lymphozyten produzieren vor allem Interleukin 4, Monozyten vor allem Interleukin 1.

Wird für eine Aktivierung der PBMC auf eine vorab erfolgende Stimulierung im wesentlichen nur der T-Lymphozyten zurückgegriffen und stimuliert man diese auf eine der vorstehend erläuterten Weisen außer durch allogene oder xenogene Zellen, so enthält diese Mischung APC, die nur "Problemproteine bzw. -peptide" des Patienten präsentieren, so daß sich die Immunreaktion der PBMC bereits in diesem Stadium gegen diese "Problemproteine bzw. -peptide" richten kann. Dies beruht, wie bereits erläutert, darauf, daß die in der Zellkultur für die T-Zellstimulierung in der Regel gleichzeitig anwesenden anderen PBMC-Zelltypen durch direkten Zellkontakt mit den T-Lymphozyten und/oder Zytokinproduktion durch die stimulierten T-Lymphozyten eine Stimulierung erfahren, die neben einer eigenen Zytokinantwort auch die Präsentation derartiger "Problemproteine bzw. -peptide" durch die anwesenden APC bewirken kann.

Bei einer Verwendung allogener, also fremder Zellen für die Stimulierung der PBMC wird zwar eine tiefgreifende Stimulierung erzielt, jedoch präsentieren die APC unter diesen Umständen in der Regel vor allem allogene Proteine bzw. Peptide und nicht die Problemkeime bzw. die daraus produzierten Peptide, die in den Zellen des Patienten vorliegen und gegen die man eine Immunantwort stimulieren möchte.

Andererseits werden auch hier T-Lymphozyten aus der PBMC-Präparation des Patienten stimuliert und von den stimulierten T-Lymphozyten Zytokine produziert. Die stimulierten oder aktivierten T-Lymphozyten aktivieren ihrerseits über direkten Zellkontakt und/oder produzierte und sezernierte Zytokine die anderen PBMC-Zelltypen aus der PBMC-Präparation des Patienten und führen so u.a. zu einer Präsentation der in den Zellen des Patienten vorliegenden "Problemproteine bzw. -peptide" durch APC gegenüber den T-Lymphozyten sowie weiteren Stimulierungen von Immunzellen. Darüberhinaus können auch weitere T-Lymphozyten über eine direkte Aktivierung ihrer αβ-T-Zellrezeptoren stimuliert werden, indem sich an diese die genannten, von den APC des Patienten im korrekten, d.h. autologen HLA- oder MHC-Kontext präsentierten "Problempeptide" des Patienten binden.

Ein Zusatz von Interferonen und auch anderen Zytokinen zur Zellkultur für die Stimulierung von T-Lymphozyten oder "aller" PBMC beeinflußt die Expansion und die Zusammensetzung der entstehenden Lymphozyten-Subpopulationen. U.U. werden auch antivirale Wirkungen derartiger Substanzen gegenüber in der Zellkultur - und bei dem zu behandelnden Patienten - vorhandenen viralen Pathogenen genutzt. Vermutlich wirken Interleukin 2 und Interferon γ synergistisch auf die vermehrte Entstehung von T-Helfer (TH) 1-Zellen. Von Interferon α und Interferon β sind derzeit nur antivirale Effekte bekannt; auch Interferon γ bietet sich wegen seiner zusätzlichen antiviralen Wirkung diesbezüglich an.

Die Auswahl derartiger Zusätze wird in der Regel nach Verträglichkeitskriterien, insbesondere bei Interferon γ, und ggf. auch nach der Virusspezifität des jeweiligen Interferons getroffen. Eine derartige gezielte Auswahl oder auch ein bewußter vollständiger Verzicht auf derartige Zusätze kann insbesondere bei der Behandlung von Patienten von Bedeutung sein, bei denen mehrere Krankheitsursachen zusammentreffen. Für Interferon α wird eine Wirksamkeit gegen Epstein-Barr-Virus berichtet. Auch auf dieser Ebene kann somit eine bei einem Patienten vorhandene Infektion durch ein Virus, das mit der zu behandelnden Erkrankung in Verbindung stehen könnte, wie dies z.B. für Epstein-Barr-Virus und Schizophrenie vermutet wird, berücksichtigt werden.

Es ist gleichfalls eine Behandlung der genannten Zellkulturen mittels mehrerer Zytokine und der mit der Virusreplikation interferierenden Zytokine, den Interferonen, gleichzeitig oder zeitlich nacheinander erfolgend möglich. Beispielsweise kann zur Erzeugung stimulierter T-Lymphozyten nach einer Inkubation der stimulierten Zellen mit Interleukin-2 eine Behandlung der Zellen mit Interferon γ oder Interferon α erfolgen.

Wie bereits angesprochen, weisen die gemäß einer speziellen Ausführungsform der Erfindung mittels des "cascade priming"-Verfahrens stimulierten PBMC gegenüber den "ein- und "zweistufig" stimulierten Zellen eine deutlich gesteigerte Wirksamkeit bei der erfindungsgemäßen Verwendung zur Behandlung von mit dem Gehirn assoziierten Erkrankungen, Störungen und Schädigungen auf. Bei der Erklärung dieses Phänomens wird u.a. davon ausgegangen, daß bei Verwendung von PBMC für die primäre Stimulierung die Stimulierung der für den weiteren Inkubationsschritt zugesetzten naiven Zellen ganz überwiegend über die aktivierten APC in der primär stimulierten Zellpopulation erfolgt, d.h. hochspezifisch über den T-Zell-Rezeptor der T-Lymphozyten (CD4-Helfer- und CD8-Killerzellen) in der naiven PBMC-Population. Je nach Vorbehandlung zur primären Stimulierung der Ausgangszellpopulation können den in dem weiteren Schritt mit der naiven PBMC-Population zugesetzten T-Zellen somit von den primär stimulierten APC unterschiedliche Peptide präsentiert werden.

Eine Hypothese zur Erklärung der deutlich erhöhten Wirksamkeit von "cascade primed" Zellen beruht darauf, daß die aus dem "cascade priming" resultierende Zellpopulation neben spezifisch angereicherten aktivierten Lymphozyten (B- und T-Zellen, vermutlich auch NK-Zellen des α/β- oder γ/δ-Rezeptortyps) überwiegend aus Gedächtniszellen, und zwar vor allem aus T-Gedächtniszellen besteht. Die immunologischen und therapeutischen Vorteile von Gedächtniszellen sind bekanntermaßen ihre Fein-Spezifität und beschleunigte Reaktivierung. Diese Gedächtniszellen benötigen folglich nur eine Minimalstimulierung zur Entfaltung ihrer vollen Aktivität. Es wird vermutet, daß "cascade primed" Zellen mit intrazellulären Mikroorganismen infizierte Zellen besonders effizient erkennen und "behandeln", d.h. pathogenfrei machen können.

Stimulierte T-Lymphozyten oder PBMC können beispielsweise in einer Dosismenge von 0,5-30 x 10⁶ stimulierten Zellen pro Injektion eingesetzt werden. Die Menge injizierter Zellen kann in Abhängigkeit vom Lebensalter, Körpergewicht und/oder von möglichen Nebenerkrankungen des Patienten angepaßt werden. Es ist möglich, die injizierte Zellmenge mit zunehmender Behandlungsdauer zu erhöhen oder ggf. auch zu senken. In der Regel liegt die Zellmenge pro Injektion unter 30 x 10⁶ aktivierten PBMC. Bevorzugt ist derzeit die Verabreichung von Mengen von 10-20 x 10⁶ Zellen pro Injektion an erwachsene Patienten und im Falle von übergewichtigen Patienten von 20-25 x 10⁶ Zellen pro Injektion. Oberhalb einer Konzentration von 50 x 10⁶ aktivierten PBMC ist mit überaus unangenehmen Nebenwirkungen zu rechnen, die vor allem durch CD4-Lymphozyten, die viele physiologisch hochaktive Verbindungen sezernieren können, hervorgerufen werden.

Aufgrund ihrer deutlich höheren Aktivität werden von den speziellen "cascade primed" Zellen in der Regel nur 1/5 bis 1/10 der ansonsten therapeutisch eingesetzten Zellmenge, also zwischen ungefähr 1 und 6 x 10⁶ Zellen eingesetzt.

Die Injektionen können in unterschiedlichen Zeitabständen, wie wöchentlich, alle drei bis vier Wochen oder mit noch längeren Zeitabständen, verabreicht werden. Die Häufigkeit der Injektionen kann mit zunehmender Dauer der Behandlung erhöht oder verringert werden.

Nach der Injektion von aktivierten Zellen können diese im Patienten mit sehr geringen Mengen von Zytokinen, vorzugsweise IL-2, reaktiviert werden. Bei IL-2 können sogar 10000 I-E, meist 25000-50000 (bis 150000) I-E ausreichen, um nach 24 bis 48 h, aber auch nach einigen Tagen die injizierten Zellen wieder zu reaktivieren. Die Zytokine werden vorzugsweise intradermal oder intramuskulär und in der Regel in einer Dosis verabreicht, können aber auch intravenös verabreicht werden.

Die Injektionen werden beispielsweise intradermal in den Arm verabreicht. Ein Teil der Injektion kann dabei alternativ in den Muskel infiltriert werden. Es ist jedoch gleichfalls möglich, die Injektionen an anderen Körperstellen intradermal und/oder intramuskulär zu verabreichen.

In einzelnen Fällen erlebten Patienten, denen "cascade primed" Zellen verabreicht wurden, kurze Zeit nach der Verabreichung eine kurzzeitige melancholische Episode, die jedoch nicht als sehr belastend empfunden wurde und rasch verging. Zur Linderung oder Vermeidung derartiger Episoden können in einer bevorzugten Ausführungsform der Erfindung in Zusammenhang mit der Verabreichung von "cascade primed" Zellen zusätzlich CD3-stimulierte Zellen verabreicht werden. Diese werden bevorzugt in eine andere Körperstelle als die "cascade primed" Zellen verabreicht. Eine Verabreichung von 10 bis 30 Millionen, bevorzugt 15 bis 25 Millionen CD3-stimulierten Zellen lieferte bislang gute Ergebnisse. Wie bei derartigen Zellen üblich, erfolgt die Verabreichung bevorzugt über eine intrakutane und/oder intramuskuläre Injektion, z.B. zu 2/3 intrakutan und zu 1/3 intramuskulär.

Die Behandlung der genannten Erkrankungen, Störungen oder Schädigungen durch die erfindungsgemäße Verwendung von aktivierten PBMC kann auch zusätzlich zu andersartiger, medikamentöser Therapie erfolgen.

So können aktivierte PBMC zur Behandlung von manisch-depressiver Krankheit zusätzlich zu üblichen Antidepressiva, wie Lithiumpräparaten, Melperon und/oder Chloprothixen, verabreicht werden. Zur Behandlung von Schizophrenie können erfindungsgemäß aktivierte PBMC zusätzlich zu einer für die Behandlung von Schizophrenie üblichen Medikamententherapie, z.B. mit Clozapin oder Fluphenazindecanoat, und zur Behandlung von Parkinson-Syndrom zusätzlich zu einer für die Behandlung von Parkinson-Syndrom üblichen Medikamententherapie, wie mittels DOPA, Bromocriptin u.s.w, eingesetzt werden.

Zur Verringerung insbesondere bakterieller Titer kann gleichzeitig auch eine Antibiotikatherapie durchgeführt werden. Hierbei können übliche Antibiotika eingesetzt werden, die ggf. aufgrund spezifischer Wirksamkeit gegen bestimmte bakterielle Erreger ausgewählt werden. Ein Beispiel hierfür ist Azithromycin, das Wirksamkeit insbesondere gegen Infektionen durch Chlamydien aufweist.

Wie bereits angesprochen, wird ein zusätzlicher Einsatz allogener transplantierter Zellen, z.B. fötaler Neurozyten, wie sie für eine "vital cell transplant therapy" eingesetzt werden, als nicht notwendig angesehen und daher grundsätzlich nicht in Betracht gezogen. Jedoch soll nicht ausgeschlossen werden, in Einzelfällen, z.B. in Zusammenhang mit auf spezielle Weise stimulierten PBMC, und insbesondere den sogenannten "cascade primed" PBMC, derartige allogene Zellen als Transplantat zusätzlich zu den auf diese spezielle Weise stimulierten PBMC einem Patienten zu verabreichen.

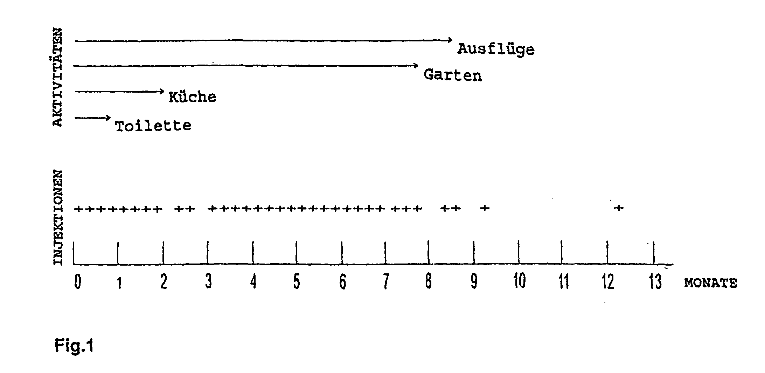

Die beigefügte Figur 1 zeigt schematisch die Korrelation bestimmter Aktivitäten eines an manisch-depressiver Krankheit leidenden Patienten mit der Anzahl von Injektionen aktivierter PBMC (vgl. Beispiel 2.1). Vor Beginn der Behandlung war der bettlägerige Patient unfähig, ohne fremde Hilfe zu laufen. Nach einigen Injektionen kam der Patient selbständig aus der Toilette zurück, ging später selbständig in die Küche und zurück und erweiterte schrittweise seinen Aktivitätsradius. Parallel hierzu wurde ein zunehmendes Interesse an sozialen Kontakten festgestellt.

### BEISPIELE

### 1. Verfahren zur PBMC-Isolierung und -Stimulierung

Mononukleäre Zellen des peripheren Bluts (PBMC) wurden aus heparinisierten Blutproben (50 Einheiten Heparin novo/ml Blut; Heparin novo: NOVO NORDISK, Mainz, DE) oder aus defibriniertem Blut über einen Ficoll-Hypaque-Gradienten (Pharmacia, Freiburg, DE) aufgereinigt, nachdem sie im Verhältnis 1:1 mit pyrogenfreier phosphatgepufferter physiologischer Kochsalzlösung (PBS) oder mit NaHCO₃ gepuffertem RPMI-1640-Medium verdünnt worden waren. Die Zellen wurden in einer befeuchteten Inkubationsvorrichtung unter Einsatz optimaler Kulturbedingungen für humane Lymphozytenzellkulturen (Wank, R., Tissue Antigens 1982: 19, 329-339) inkubiert. Kurz zusammengefaßt wurden die Zellen bei 38,5°C unter Einsatz von 6,5% CO₂ entweder mit allogenen Zellen oder auf mit anti-CD3-Antikörpern beschichteten Costar-Mikrotiterplatten mit 24 Vertiefungen inkubiert. Die PBMC wurden auf drei Weisen stimuliert: mit allogenen Zellen (1.1.), mit anti-CD3-Antikörpern (1.2.) sowie mit anti-CD3-Antikörpern unter Zusatz von IFNα oder IFNγ (1.3), und mit IL-2 expandiert.

### 1.1. Stimulierung mit allogenen PBMC

PBMC des Patienten wurden mit bestrahlten allogenen Zellen (s.u.) im Verhältnis 1:1 unter Verwendung von 2 x 10⁶ Zellen/ml in mit NaHCO₃ gepuffertem RPMI-1640-Kulturmedium, ergänzt mit 10% fötalem Kälberserum (FCS; Hyclone), stimuliert. Die alloaktivierten Zellen des Patienten wurden nach 5 Tagen mit 20 I.E. Interleukin 2 (Proleukin, Euro-Cetus, Ratingen, DE) pro ml Kulturmedium 48 h lang expandiert. Die Zellen wurden geerntet, dreimal mit PBS gewaschen und in einer Konzentration von 10 x 10⁶ Zellen/ml verwendet oder für eine spätere Verwendung eingefroren aufbewahrt. Verfahren für eine derartige Aufbewahrung ("Cryopreservation") sind beispielsweise beschrieben in Schendel, D.J., Maget, B., Falk, S.C., und Wank, R., Human CD8+ T lymphocytes, in: Immunology Methods Manual, Academic Press Ltd., 1997, Hrsg. Rammensee, 9.8: 669-690. Kurz zusammengefaßt, wurde auf 4°C gekühltes DMSO dem vorstehend beschriebenen Kulturmedium, das mit 50% FCS (Hyclone) supplementiert war, bis zu einer Endkonzentration von 10% zugesetzt. Die Proben wurden in einer Tiefkühltruhe (Revco) bei -80°C eingefroren und in der Gasphase eines Tanks mit flüssigem Stickstoff aufbewahrt.

Allogene PBMC wurden vor der Verwendung zur Stimulierung von PBMC eines Patienten mit 20 Sievert (Sv) unter Einsatz einer Caesium-Quelle mit einer Rate von 1 Sv/min bestrahlt. Allogene Spender und Patienten wurden vorher serologisch auf Antikörper gegen HIV, Hepatitis A, B, C, CMV, EBV, HSV 1 und 2, HHV 6, Chlamydia pneumoniae, psittaci, trachomatis, toxoplasmosis und Antikörper gegen Cardiolipin getestet. Spenderzellen wurden in der Gasphase über flüssigem Stickstoff 6 Monate gefroren gelagert ("Cryopreservation") und nur nach einem zweiten negativen Test auf Antikörper gegen HIV, CMV und Hepatitisviren verwendet.

Allogen stimulierte PBMC wurden in den Patientenstudien 2.1 und 2.4, hier nur zu Anfang, eingesetzt.

### 1.2. Stimulierung mit immobilisierten monoklonalen anti-CD3-Antikörpern und Expansion mit Interleukin 2

PBMC von Patienten wurden mit immobilisierten anti-CD3-Antikörpern (Orthoclone OKT 3, Cilag, Sulzbach/Taunus, DE) stimuliert und Interleukin 2 frühestens nach 2 h, spätestens nach 20 h, in der Regel nach 18 h zugegeben, eventuell auch Interferon (IFN) zugesetzt. Die Zellen wurden in vitro vier Tage aktiviert und anschließend unter Zusatz von Interleukin 2 drei Tage expandiert.

Die Immobilisierung der monoklonalen Antikörper erfolgte, wie folgt: 1 µg OKT 3-Antikörper/ml wurde in 0,05 M Boratpuffer suspendiert, der mit 1 N NaOH auf pH 8,6 eingestellt wurde. Das Gemisch wurde in 0,1 ml Aliquots/Vertiefung auf Costar-Mikrotiterplatten mit 24 Vertiefungen verteilt. Die mit Antikörper beschichteten Platten wurden bei 4°C aufbewahrt und zweimal mit 2 ml mit Phosphat gepufferter physiologischer Kochsalzlösung vor der Inkubation mit PBMC gewaschen. Die PBMC wurden vier Tage in einer befeuchteten Inkubationsvorrichtung unter Einsatz der in (1.) beschriebenen Bedingungen in einer Konzentration von 1 x 10⁶/ml inkubiert und mit 20 I.E. Interleukin 2/ml (Proleukin, EuroCetus, Ratingen, DE) supplementiert. Die Zellen wurden nach dem Ernten dreimal gewaschen.

Die CD3-Stimulierung kann auch in Kulturflaschen erfolgen. Es müssen 20 x 10⁶ Zellen pro Kulturflasche in einem Volumen von 12 ml verteilt werden, wobei die Interleukin 2-Konzentration hier auf 27 I.E./ml eingestellt wird. Die Expansion nach der jeweiligen Stimulierung in Kulturplatten oder Kulturflaschen ist methodisch gleich:
Nach dem Ernten, dreimaligem Waschen und Zählen werden die Zellen in frischem Kulturmedium resuspendiert und in Kulturflaschen (Konzentration 7 x 10⁶ in 25 ml Kulturmedium) unter Zusatz von 20 I.E. Interleukin 2 72 h amplifiziert.

### 1.3. Stimulierung mit anti-CD3-Antikörpern, Expansion mit IL-2 und Supplementierung mit Interferon γ (IFNγ) oder Interferon α (IFNα)

PBMC von Patienten wurden mit immobilisierten anti-CD3-Antikörpern stimuliert und mit Interleukin 2 expandiert, wie in 1.2. beschrieben. 5 ng IFNγ/ml (Polyferon, Rentschler, Laupheim, DE) wurden nach 18 h zugesetzt, um die Immunantwort in Richtung eines Th-Typs zu beeinflussen. IFNα wurde den stimulierten Zellkulturen in einer Konzentration von 600 I.E./ml in separaten Kulturen zugeetzt.

### 1.4. Herstellung von "cascade primed" Zellen

PBMC wurden gemäß den vorstehend unter 1.2. oder 1.3. angegebenen Verfahren für 3-6 h primär stimuliert. Daraufhin wurden naive PBMC im Verhältnis 1:1 in dem in Zusammenhang mit der Primärstimulierung zuletzt verwendeten Kulturmedium, z.B. RPMI-1640 einschließlich 10% Hyclone FCS, zugesetzt und die Inkubation für 4-5 Tage fortgesetzt.

### 1.5. Aufbewahrung in gefrorenem Zustand ("Cryopreservation") und Auftauen stimulierter Lymphozyten

Alle Zellen wurden geerntet und gefroren in Aliquots von 5 x, 10 x und 20 x 10⁶ Zellen unter Verwendung von RPMI-1640-Medium, supplementiert mit 10 - 50% fötalem Kälberserum (Hyclone), und mit 10% DMSO versetzt aufbewahrt. Die Einzelheiten der Aufbewahrung und des Auftauens sind erläutert in Wank, R., Tissue Antigens 1982: 19, 329-339, und in Schendel, D.J., Maget, B., Falk, S.C., und Wank, R., Human CD8+ T lymphocytes, in: Immunology Methods Manual, Academic Press Ltd., 1997, Hrsg. Rammensee, 9.8: 669-690.

Die bei den nachfolgenden Patientenstudien angegebenen Zellzahlen sind mit einer Genauigkeit von ± 20% angegeben. Wenn man eine bestimmte Zellzahl kryopräserviert, schwankt die nach dem Auftauen rückgewinnbare Zellzahl.

### 2. Patientenstudien

Die Studien wurden an vier Patienten vorgenommen, die sämtlich über mindestens die letzten fünf Jahre vor Beginn der Studie an einer chronischen Geisteskrankheit litten. Die Diagnose jedes Patienten wurde mindestens einmal in einer psychiatrischen Klinik eines Universitätskrankenhauses bestätigt. Die Patienten mit manisch-depressiver Krankheit, Schizophrenie und Autismus wurden gemäß den DMS-IIIR-Kriterien diagnostiziert.

Bei sämtlichen Patienten wurde die verordnete psychiatrische Medikamentierung nicht verändert, es sei denn, der verantwortliche Psychiater schlug eine Änderung vor oder stimmte einer solchen zu. Im Prinzip wurde der Behandlung mit aktivierten PBMC die Rolle einer adjuvanten Therapie zugewiesen.

### 2.1. Behandlung eines Patienten mit manisch-depressiver Krankheit

Der erste Patient war zu Beginn der adoptiven Immuntherapie im Jahre 1992 71 Jahre alt. Eine manische Depression wurde 1955 diagnostiziert und mit gewissem Erfolg mit Lithiumpräparaten, Anafranil und Clomipramin behandelt. In 1987 wurde der Patient aufgrund von Symptomen einer Lithium-Vergiftung in ein Krankenhaus eingewiesen. 1990 konnte der Patient Orte in seiner Heimatstadt, die ihm wohlbekannt waren, nicht mehr erkennen, sich nicht an Namen erinnern und hatte zunehmende Schwierigkeiten, seinen Namen zu schreiben. Sein Gleichgewichtssinn war tiefgreifend gestört. Der Verdacht auf einen Infarkt im Bereich der Basalganglien konnte durch eine Computertomographie des Gehirns nicht bestätigt werden. Die Enddiagnose einer Untersuchung in einer neurologischen Abteilung eines Krankenhauses in 1990 lautete auf eine manisch-depressive Krankheit, jetzt mit vorherrschender schwerer Depression, Hypertonie und Ischämie der rechten Arteria cerebri posterior, Multiinfarktdemenz, kompensiertes Nierenversagen, Glaukome an beiden Augen, Basaliom im Bereich des Thorax und Parkinson-Syndrom mit Schwerpunkt auf der linken Seite mit Tremor im Ruhezustand und Rigor. Es wurde angenommen, daß das Parkinson-Syndrom arzneimittelinduziert war. Die Lithiumtherapie wurde auch aufgrund einer persistierenden Beeinträchtigung der Harnkonzentration abgebrochen. Der Patient erhielt nachfolgend Melperon und Chloprothixen bis zu seinem Tod in 1993.

Familiengeschichte: Die Mutter und zwei der vier Geschwister des Patienten waren mehrfach in psychiatrischer Behandlung.

Behandlung des Patienten: Der Patient erhielt 33 Injektionen von allogen stimulierten Zellen (siehe 1.1) innerhalb von 51 Wochen, wobei Injektionen in den Monaten 1 bis 9 im wesentlichen wöchentlich verabreicht wurden (siehe Fig. 1). Einige Male wurden die Zeitabstände zwischen zwei aufeinanderfolgenden Injektionen aufgrund eines Aufenthaltes des Patienten in einem Pflegeheim auf zwei Wochen und einmal auf drei Wochen erhöht. Die Injektionen umfaßten jeweils 20 x 10⁶ allogen aktivierte PMBC, die intradermal in drei Portionen an drei unterschiedlichen Stellen des Unterarms verabreicht wurden; eine Hälfte der dritten Teilinjektion wurden in den Muskel infiltriert. Bei unklaren Beschwerden, die in dem Zeitraum von 2-6 Tagen nach der Injektion auftraten, wurde vorsichtshalber bei der darauffolgenden Zellbehandlung die Dosis auf 10 x 10⁶ Zellen reduziert. Nach der fünfzehnten Injektion wurde die Dosierung beider antipsychotischer Arzneimittel Melperon-HCl und Chlorprothixen um die Hälfte reduziert.

Zusammenfassung der erzielten Ergebnisse bei dem ersten Patienten:

Die manisch-depressive Erkrankung wurde bei diesem Patienten im Alter von 37 Jahren diagnostiziert und mehrere Jahrzehnte lang erfolgreich mit mehreren Antidepressiva, einschließlich Lithium, behandelt. In den letzten Jahren mußte diese Therapie abgesetzt werden, da der Patient auf diese Therapie nicht mehr sehr gut ansprach und Nierenprobleme entwickelte.

Zu Beginn der adoptiven Immuntherapie war der Patient bettlägerig. Für die Einnahme der Mahlzeiten mußte er mit einem Rollstuhl zum Tisch gefahren werden. Das arzneimittelinduzierte Parkinson-Syndrom trug sicherlich zu diesem Zustand bei, jedoch befand sich der Patient gleichfalls in einem Zustand schwerer Depression. Im Verlauf der adoptiven Immuntherapie konnte eine stetige Zunahme des Selbstvertrauens beobachtet werden, die zu einem erhöhten Aktionsbereich führte (Fig. 1). Dies war zu einem späteren Zeitpunkt unabhängig von der Möglichkeit, sich diesen Raum zu Fuß zu erschließen, d.h. der Patient nahm Einladungen an und akzeptierte Ausflüge mit dem Auto. Bereits nach der ersten Injektion konnten Veränderungen im Sozialverhalten des Patienten festgestellt werden. Im Gegensatz zu dem zumeist desinteressierten oder negativen Verhalten des Patienten vor Beginn der Immuntherapie zeigte dieser nun Anerkennung für die Pflegebemühungen seiner Frau und war besorgt über ihr anstrengendes Leben. Diese positive emotionale Entwicklung wie auch eine Verbesserung der physischen Leistungsfähigkeit hielten an.

Das wahrscheinlich arzneimittelinduzierte Parkinson-Syndrom verhinderte sicherlich Unternehmungen, die einen guten physischen Zustand erforderten. Jedoch begann der Patient selbsttätig zur Toilette und später zum Garten zu gehen und von diesen zurückzukehren. Er entwickelte ein Interesse an Ausflügen mit dem Auto und fand Gefallen daran, in der Kirche während der Hochzeit seiner Tochter anwesend zu sein, obwohl er in einem Rollstuhl transportiert werden mußte. Er überraschte seine Frau immer wieder mit neuen Initiativen, wie dem Inkontakttreten mit Verwandten oder mit Gehen ohne Hilfe.

Bedauerlicherweise verstarb der Patient nach der angegebenen Therapie in einem Pflegeheim aus Gründen, die zu der Immuntherapie nicht in Beziehung standen.

### 2.2. Behandlung eines an Autismus leidenden Patienten

Der zweite Patient war zu Beginn der adoptiven Immuntherapie im Jahre 1993 9 Jahre alt. Autismus wurde im Alter von 28 Monaten diagnostiziert (ICD 290). Wie üblich wurden keine Anzeichen einer abnormalen Entwicklung bei der Geburt und innerhalb der ersten zwei Lebensjahre festgestellt. Innerhalb der letzten fünf Jahre vor Beginn der Immuntherapie war keine bestimmte Arzneimitteltherapie verabreicht worden. Der Patient wurde von seiner Mutter auf einer weizenfreien Diät gehalten und besuchte eine Sonderschule.

Familiengeschichte: Von Seiten der Mutter waren keine Familienmitglieder mit psychischen Problemen bekannt. Die Familiengeschichte des Vaters war nicht ermittelbar.

Behandlung des Patienten: Der Patient erhielt insgesamt 29 Injektionen mit CD3-aktivierten Lymphozyten von November 1993 bis November 1996. Die Behandlungen erfolgten in der Regel alle drei bis vier Wochen, wobei in Einzelfällen der Zeitabstand zwischen zwei aufeinanderfolgenden Injektionen auch bis zu zwei Monate betragen konnte. In 1996 wurden die Zeitabstände zwischen aufeinanderfolgenden Injektionen auf ca. 2 Monate erhöht. Die injizierte Zellzahl betrug in 1993 und 1994 jeweils 1,5 x 10⁶, bei der zweiten Injektion in einem Einzelfall 3 x 10⁶ aktivierte Zellen, und wurde schrittweise in 1995 auf 5 x 10⁶ aktivierte Zellen und in 1996 auf 8 x 10⁶ aktivierte Zellen pro Injektion erhöht. Die Injektionen erfolgten in den Arm.

### Ergänzende Laboruntersuchungen an dem zweiten Patienten:

Es wurde ein Antikörper-Screening auf Antikörper gegen Viren und gegen Chlamydien ausgeführt. Eine serologische Analyse zeigte, daß der Patient Antikörpertiter weder gegen das Hepatitisvirus A, B oder C noch gegen CMV aufwies. Er hatte niedrige IgG-Antikörpertiter gegen HSV (0,5 Einheiten/ml) und HHV6 (1:32). Die EBV-Serologie ergab IgG-Antikörper gegen EBV-VCA (EBV virales Capsidantigen; 1:64), EA (EBV-Frühantigen; 1:8) und EBNA (Epstein-Barr nukleäres Antigen). Es konnten keine IgM-Antikörper gegen EBV-VCA nachgewiesen werden, so daß eine frische EBV-Injektion ausgeschlossen werden konnte. Der Patient zeigte substanziell erhöhte IgG-Antikörpertiter gegen Chlamydia pneumoniae (1:256), aber nicht gegen Chlamydia trachomatis. Eine Analyse der Antikörper der Mutter gegen Chlamydien ergab IgG-Antikörper gegen Chlamydia pneumoniae (1:256) und Chlamydia trachomatis (1:64).

Die adoptive Immuntherapie mußte einmal für eine längere Zeitdauer aufgrund einer Grippeerkrankung oder grippeähnlichen Infektion unterbrochen werden. Die grippeähnliche Infektion könnte durch die chronische Infektion mit Chlamydia pneumoniae hervorgerufen worden sein.

Zusammenfassung der erzielten Ergebnisse bei dem zweiten Patienten:

Der Patient war aggressiv und hatte typische Wutanfälle bei den ersten drei Begegnungen. Während der ersten Blutabnahme und der ersten Injektion mußte der Patient von mehreren Personen festgehalten werden. Dies änderte sich im Laufe weiterer Injektionen und der Patient erinnerte sogar seine Mutter daran, weitere Behandlungstermine wahrzunehmen.

Die Mutter berichtete, daß der Patient bereits nach der ersten Injektion begann, die Worte "ich" und "mir" häufiger zu verwenden und über sich nicht mehr als dritte Person sprach. Sie wie auch Nachbarn des Patienten stellten eine verbesserte Artikulation des Patienten fest. Die Verbesserung der Artikulation nahm zwar nach drei Wochen ab, jedoch blieb der Zustand besser als vor der Therapie. Im Laufe der Therapie begann der Patient, das erste Mal emotionale Anteilnahme an der Befindlichkeit seiner Mutter zu zeigen. Zusätzlich nahm die Kontaktfähigkeit gegenüber dritten Personen z.B. während der Behandlungstermine oder mit seinen Schulkameraden zu. Die Schullehrer stellten bemerkenswerte Fortschritte der Lernfähigkeit und der Fähigkeit, sich auf eine Aufgabe zu konzentrieren, fest.

6 Monate nach der letzten Injektion wurde von der Mutter des Patienten berichtet, daß die positive Entwicklung des Patienten sich fortgesetzt habe. Bemerkenswert sei insbesondere das gute Verhältnis zu den Schulkameraden, die keine plötzlichen Angriffe des Patienten mehr befürchten müßten, und auch die schulischen Leistungen seien zufriedenstellend.

### 2.3 Behandlung eines an paranoider Schizophrenie leidenden Patienten

Der dritte, weibliche Patient war zu Beginn der adoptiven Immuntherapie im Jahre 1996 23 Jahre alt. Paranoide Schizophrenie mit akustischen Halluzinationen wurde 1993 durch einen Psychologen und einen Psychiater diagnostiziert und 1995 in der psychiatrischen Klinik eines Universitätskrankenhauses bestätigt (ICD9 Nr. 295.3).

Die Patientin litt unter akustischen Halluzinationen mit einem fortlaufenden Kommentar durch einen der Elternteile oder den Psychologen, der sämtliche Aktionen der Patientin in Frage stellte. Die Patientin war unfähig, sich auf irgendeine Aufgabe zu konzentrieren, was zum Abbruch des Studiums geführt hatte. Darüberhinaus traten Wutanfälle auf. Die Patientin fühlte sich zusätzlich durch eine tiefe Müdigkeit beeinträchtigt, zeigte kaum eine spontane Bewegung und die Stimme erschien monoton.

Eine Behandlung mit 250 mg Clozapin/Tag, unterstützt von kognitivem Training und Beschäftigungstherapie, verbesserte die Angstzustände und kognitiven Fähigkeiten. Die Wutanfälle verschwanden. Die akustischen Halluzinationen blieben bestehen, waren jedoch seltener und schwächer.

Familiengeschichte: Großeltern und Eltern schienen gesund zu sein, obwohl die Mutter einmal aufgrund einer depressiven Störung behandelt worden war, die jedoch bereits seit einiger Zeit keine medikamentöse Behandlung mehr erforderte.

Behandlung der Patientin: Die Patientin erhielt insgesamt 32 Injektionen mit CD3-aktivierten Lymphozyten von Dezember 1995 bis Januar 1997. Zu Beginn wurden ungefähr 10 x 10⁶ aktivierte Zellen alle 14 Tage injiziert. Nach drei Monaten wurde die Injektionshäufigkeit auf einmal wöchentlich und die Zelldosis auf 15 x 10⁶ Zellen erhöht. Nach einem Jahr wurden die Injektionen mit jeweils 10-12 x 10⁶ Zellen einer Kombination aus CD3-aktivierten Zellen mit und ohne IFNγ-Kulturzusatz fortgesetzt.

Ergänzende Laboruntersuchungen an der dritten Patientin:

Die Laboruntersuchungen ergaben normalen Thyroid-, Vitamin B12-und Folsäuremetabolismus wie auch ein normales EKG und Schädel-MRT. Geringfügige Abweichungen im EEG waren höchstwahrscheinlich arzneimittelinduziert.

Es wurden positive Antikörper-IgG-Titer gegen EBV, CMV, HHV6 und HSV, jedoch keine gegen Hepatitisvirus A, B oder C festgestellt. Die serologische Untersuchung auf Chlamydien zeigte eine positive Komplementbindungsreaktion, jedoch negative IgA-und IgG-Titer. In der nachfolgend gezeigten Tabelle sind nur Chlamydien- und EBV-Antikörpertiter gezeigt. Da die Patientin im Laufe der Jahre Kontakt zu vielen verschiedenen Haustieren gehabt hatte, war es überraschend, daß dies sich nicht in positiven Antikörpertitern gegen Chlamydien oder Toxoplasma niederschlug. Aufgrund dessen, der positiven Komplementbindungsreaktion und eines chronischen, geringgradigen Vaginalausflusses seit der Kindheit wurde ein PCR-Screening durch einen Gynäkologen trotz negativer Immunfluoreszenzanfärbung veranlaßt. Die PCR-Reaktion zeigte eine für Chlamydia trachomatis spezifische positive Reaktion. Dies erklärt die positive Komplementbindungsreaktion gegenüber Chlamydienantigenen und zeigt eine persistierende Chlamydieninfektion in Verbindung mit einem Fehlen einer signifikanten Antikörperantwort an.

Zusammenfassung der erzielten Ergebnisse bei der dritten Patientin:

Wie bereits erläutert, litt die Patientin zu Beginn der Behandlung unter akustischen Halluzinationen, Unfähigkeit, sich auf irgendeine Aufgabe zu konzentrieren, Wutanfällen und tiefer Müdigkeit. Sie zeigte kaum eine spontane Bewegung und die Stimme erschien monoton. Die Patientin unterzog sich zu Beginn der adoptiven Immuntherapie bereits ein Jahr lang einer Clozapin-Therapie und die paranoiden Symptome waren gut kontrolliert.

Vor Beginn der Behandlung verließ die Patientin das Bett erst am Nachmittag. Nach zwei Monaten adoptiver Immuntherapie stand die Patienten regelmäßig um 7 Uhr morgens auf, um eine Sekretärinnenschule zu besuchen. Es gab nur wenige Fehlzeiten mit einer Häufigkeit von weniger als einmal pro Monat, üblicherweise Montags. Die Diskussionen mit der Patientin ergaben, daß die positiven Wirkungen der Immuntherapie die ersten drei Tage optimal waren und dann abzunehmen begannen. Dementsprechend wurde zur Überwindung der Probleme an den Montagen beschlossen, die Injektionen jeweils freitags zu verabreichen. Die Ausbildung zur Sekretärin wurde erfolgreich abgeschlossen und es folgte eine gleichfalls zufriedenstellende Tätigkeit in einem Sekretariat einer Anwaltskanzlei. Um der Patientin eine ruhigere Arbeitsumgebung als in einem Sekretariat zu ermöglichen, wurde in der Folge ein Stellungswechsel realisiert. Der Patientin geht es seither sehr gut und die Therapie wird auf wöchentlicher Basis fortgesetzt.

Die adoptive Immuntherapie konnte die Halluzinationen nicht vollständig beseitigen, aber sie wurden sehr selten und freundlich. Die Hauptwirkung der adoptiven Immuntherapie war die Reaktivierung der Fähigkeit für ein soziales Leben und eine Wiedergewinnung der emotionalen Bandbreite bei sozialen Kontakten. Die Patientin war nicht nur dazu in der Lage, früh aufzustehen und erfolgreich eine Berufsschule zu besuchen, sondern auch daran Gefallen zu finden.

### 2.4. Behandlung eines weiteren an paranoider Schizophrenie leidenden Patienten

Der vierte, männliche Patient war zu Beginn der adoptiven Immuntherapie im Jahre 1994 31 Jahre alt. Paranoide Schizophrenie wurde 1983 diagnostiziert. Der Patient war aufgrund psychotischer Attacken in mehrere psychiatrischen Kliniken in Deutschland und einmal in den U.S.A. für insgesamt 2 Jahre stationär eingewiesen worden. Die Berichte betonen die Beschäftigung mit akustischen Halluzinationen und eine Bewahrung kognitiver Funktionen und von Affekt, so daß die diagnostischen Kriterien für paranoide Schizophrenie erfüllt waren (ICD 295.30).

Die ersten paranoiden Symptome des Patienten wurden im Alter von 20 Jahren festgestellt. Zwei Jahre später verließ er das Bett nicht mehr. Ihn beschäftigten bestimmte Körperempfindungen stark; so war er der Annahme, eine offene Wunde, ein Loch im Kopf oder eine nicht geschlossene Stelle in seinem Thorax zu haben. Auch gab er eine telepathische Verbindung mit einem buddhistischen Priester an, der ihm Anweisungen bezüglich seines Verhaltens geben würde.

Zu Beginn der adoptiven Immuntherapie war die psychiatrische Medikation Fluphenazindecanoat (Lyogen depot 50) i.m., wobei vierzehntägig eine Ampulle verabreicht wurde.

Familiengeschichte: Gemäß einem Bericht des Patienten leidet eine ältere Schwester desselben wiederholt unter depressiven Symptomen.

Behandlung des Patienten: Der Patient erhielt insgesamt 104 Injektionen von Februar 1994 bis Januar 1997. Zu Beginn der Behandlung im Februar 1994 wurden 12 x 10⁶, danach 6 x 10⁶ allogen stimulierte Zellen (siehe 1.1.) in den Unterarm injiziert. Trotz positiver Reaktionen aufgrund der alloaktivierten autologen Zellen, wie in 1.1. beschrieben, wurde das Aktivierungsverfahren geändert und immobilisierte anti-CD3-Antikörper für die Aktivierung, wie in 1.2. beschrieben, verwendet. Der Grund dafür bestand darin, daß der Patient durch die allogen stimulierten Zellen zwar zunehmende Aktivierung und zunehmendes Wohlbefinden, aber auch eine Zunahme "psychotischer Stimmungen" erfuhr. Anti-CD3-aktivierte Zellen allein hatten jedoch nur eine geringfügige und kurzfristige therapeutische Wirkung. Der Patient erhielt nachfolgend sowohl anti-CD3-aktivierte Zellen, wie in 1.2. beschrieben, als auch anti-CD3-aktivierte Zellen, die mit IFNγ während der in vitro-Kultivierung supplementiert worden waren, gemäß 1.3. Jede Zellpräparation wurde entweder in den rechten oder in den linken Arm injiziert. Bei CD3-aktivierten Zellen wurde durch Zugabe von IFNγ zu den Zellkulturen ein länger andauernder und stärkerer Effekt festgestellt. Das örtliche Anschwellen nach der Injektion war stärker als bei Zellen allein mit CD3-Aktivierung, so daß die Dosis injizierter Zellen bei einer "mittleren" Dosis von 10 x 10⁶ Zellen/Injektion alle vierzehn Tage in 1995 gehalten wurde. Im darauffolgenden Jahr wurde zu einer wöchentlichen Verabreichung übergegangen. Mit der Abnahme lokaler Reaktionen im Oktober 1996 wurde das wöchentliche Verabreichungsschema weitergeführt und die Zelldosis schrittweise auf 20 x 10⁶ Zellen/Injektion erhöht.

Ergänzende Laboruntersuchungen an dem vierten Patienten:

Der Patient hatte Antikörpertiter, die eine aktive Infektion mit Chlamydia pneumoniae wie auch mit Chlamydia trachomatis anzeigten. Sowohl die IgA- als auch die IgG-Titer waren erhöht (siehe Tabelle 1).

Zusammenfassung der erzielten Ergebnisse bei dem vierten Patienten:

Bereits einen Tag nach der ersten Injektion fühlte sich der Patient besser, "in Harmonie mit sich". Alpträume und nächtliche Schweißausbrüche verschwanden innerhalb der ersten sechs Monate der Immuntherapie. Die anderen Körperempfindungen des Patienten verschwanden während dieses Zeitraums gleichfalls oder wurden gelindert. Die telepathisch wahrgenommenen Stimmen des buddhistischen Mönches verschwanden nicht, wurden jedoch freundlicher und hielten den Patienten nicht länger von seinem künstlerischen Schaffen ab. Interessanterweise empfand der Patient eine Erhöhung seines Wohlbefindens durch das Antibiotikum Azithromycin, das aufgrund der positiven Chlamydien-Antikörpertiter verabreicht wurde.

Die Entwicklung des Patienten zu größerer Selbständigkeit wiederspiegelte sich in seinem Verhalten gegenüber den Eltern. Im ersten Jahr der Therapie nahm der Patient im Hause seiner Eltern Zuflucht in den Zeiten von Ruhelosigkeit. In 1996 tat er dies nicht mehr. Er schien daran Gefallen zu haben, allein zu leben.

Um einen möglichen "Clearing"-Effekt auf bakterielle und virale Titer durch die adoptive Immuntherapie und ggf. synergistische Effekte durch zusätzliche Antibiotikatherapie zu untersuchen, erhielt der Patient in 1994 und Anfang 1995 fünf Runden von 1,5 g Azithromycin. Es wurde ein dreitägiges Verabreichungsschema eingesetzt. Der Patient berichtete nach jeder Runde über eine Verbesserung für mehrere Tage. Aufgrund von Diarrhöe mußte die Medikation nach der fünften Runde Azithromycin beendet werden. Die adoptive Immuntherapie wurde während der Antibiotikatherapie nicht unterbrochen.

Ein Versuch mit Docyclin nach mehreren Monaten zeigte keine vergleichbare therapeutische Wirkung hinsichtlich der von dem Patienten "festgestellten" Verbesserung bei den genannten Körperempfindungen. Dies könnte bedeuten, daß die weit bessere Fähigkeit von Azithromycin, in das ZNS einzudringen, von Bedeutung sein könnte; alternativ könnte Azithromycin eine bislang unbekannte psychodynamische Wirkung aufweisen. Jedoch zeigte eine Runde mit Azithromycin zwei Jahre später nicht mehr die starken therapeutischen Wirkungen auf das geistige Wohlbefinden. Dies spricht gegen eine bedeutende primäre psychodynamische Wirkung der vorangegangenen Azithromycinmedikationen, sondern vielmehr für eine Beteiligung von Chlamydien oder anderen Azithromycin-empfindlichen Pathogenen als Faktoren am geistigen Wohlbefinden.

Es könnte eine synergistische Wirkung der adoptiven Immuntherapie und Azithromycin bestehen, da berichtet worden ist, daß Immunzellen Azithromycin aufnehmen und an den Ort der Infektion transportieren (Bergan, T. et al., Scand. J. Infect. Dis. 1992: Suppl. 83: 15-21). Antikörpertiter gegen das VCA-Antigen nahmen ab, aber IgG-Antikörper gegen das frühe Antigen (EA) des EBV, wie sie bei dem Patienten festgestellt wurden, könnten eine nach wie vor schwelende lytische Infektion anzeigen. Diese Ergebnisse zeigen eine in vivo-Wirksamkeit einer adoptiven Immuntherapie mit niedriger Dosierung über eine lange Zeitdauer, z.B. 18 Monate, um immer wiederkehrende chronische EBV-Infektionen einzuschränken.

Wie bereits ausgeführt, wurde der Patient seit fünf Jahren durch seinen psychiatrischen Arzt mit intramuskulären Injektionen von Fluphenazin behandelt. Bei Beginn der adoptiven Immuntherapie hatte der Patient seine Kurse an der Universität für mehr als 14 Monate nicht mehr besucht. Das Fluphenazin-Verabreichungsschema wurde während der ersten 18 Monate der Immuntherapie nicht geändert. In diesem Zeitraum nahm der Patient seine Studien an der Universität wieder auf und beendete dieses erfolgreich mit einem Diplom. Nach diesen Erfolgen begann der Patient bei einem neuen psychiatrischen Arzt mit einer Clozapintherapie. Clozapin linderte die verbliebenen psychotischen Episoden des Patienten, führte jedoch zu verringerter Mobilität. Der Patient klagte über schwere Glieder. Diese Nebenwirkungen von Clozapin schienen durch erhöhte Anzahlen injizierter Zellen gelindert zu werden.

Es war eine kontinuierliche allgemeine Verbesserung bei dem Patienten seit Beginn der adoptiven Immuntherapie festzustellen. Obwohl die akustischen Halluzinationen nach wie vor bestanden, wurden die Stimmen als entfernter und machtlos empfunden. Die Verbesserung spiegelt sich am besten in der erfolgreichen Karriere des Patienten in einem Universitätsstudium wieder, das er mit einem Diplom als Goldschmied beendete. Er lebt nun unabhängig in seinem Atelier, was er schätzt. Er muß auf seine Eltern nicht mehr zurückgreifen. Parallel zu dieser Entwicklung trat ein Abfall der Antikörpertiter gegen EBV-VCA und Chlamydia trachomatis zu einem beherrschteren Zustand der Infektionen auf, obwohl IgG-Antikörpertiter gegen das frühe Antigen von EBV und IgA-Antikörper gegen Chlamydia pneumoniae pathologisch bleiben (Tabelle 2).

Hinsichtlich der Wirksamkeit der verabreichten einzelnen Therapien wird gegenwärtig angenommen, daß Azithromycin dazu beitrug, die Chlamydieninfektionen zurückzudrängen, und Clozapin erfolgreich die Psychosen bekämpfte. Die Immuntherapie spielte eine Hauptrolle bei der Verringerung sowohl der lytischen EBV-Infektion als auch der Chlamydieninfektion; ferner war sie entscheidend für die Rückkehr des Patienten in das Berufsleben, indem sie die geistige Energie und Munterkeit wiederherstellte.

Zu einem späteren Zeitpunkt konnte bei diesem Patienten durch Einsatz von "cascade primed" Zellen eine weitere Besserung erzielt werden. Hierzu wurden diesem zunächst über vier Wochen hinweg jeweils einmal wöchentlich zwischen 4 und 6 Millionen "cascade primed" Zellen verabreicht. Danach wurde die Therapie mit einer einmaligen Injektion von 4 bis 6 Millionen "cascade primed" Zellen pro Monat fortgesetzt. Zur Linderung oder Vermeidung kurzzeitiger melancholischer Episoden kurz nach den Injektionen wurden dem Patienten unmittelbar in Zusammenhang mit den Injektionen zusätzlich jeweils 15 bis 25 Millionen CD-3 stimulierte Zellen durch Injektion in eine andere Stelle des Körpers verabreicht. Diese Injektion erfolgte zu 2/3 intrakutan und zu 1/3 intramuskulär. Parallel zu dieser Therapie wurde die Antibiotika-Therapie zur Verringerung der Chlamydien-Titer alle zwei bis drei Monate wiederholt.

**TABELLE 1**

| ANTIKÖRPERTITER GEGEN CHLAMYDIEN UND EPSTEIN-BARR-VIRUS BEI DEN PATIENTEN 2, 3 UND 4 | | | |
|---|---|---|---|
| Patient Diagnose Geschlecht | Patient 2 Autismus männlich | Patient 3 Schizophrenie weiblich | Patient 4 Schizophrenie männlich |
| Alter (Jahre) | 9 | 22 | 32 |
| C. pneumoniae | | | |
| IgA | nicht best. | nicht best. | 1:32 |
| IgG | 1:256¹⁾ | negativ | 1:64¹⁾ |
| | | | |

| C.trachomatis | | | |
|---|---|---|---|
| IgA | | | 1:8¹⁾ |
| IgG | negativ | negativ (PCR-positiv)²⁾ | 1:8¹⁾ |

| Epstein-Barr-Virus | | | |
|---|---|---|---|
| VCA IgM | nicht best. | negativ | negativ |
| VCA IgG | 1:64 | 1:32 | 1:512¹⁾ |
| EA IgM | nicht best. | nicht best. | negativ |
| EA IgG | 1:8 | negativ | 1:64¹⁾ |
| EBNA | schwach positiv | schwach positiv | unspezifisch |

| | | | |
|---|---|---|---|
| Die Seren für die Bestimmung der Antikörpertiter wurde vor Beginn der Immuntherapie gesammelt. ¹⁾ Antikörpertiter, die eine frische oder rekurrente Infektion anzeigen ²⁾ PCR wurde an Cervixabstrichen vorgenommen. | | | |

**TABELLE 2**

| TIERE¹⁾ DER PATIENTEN 1, 2, 3 UND 4 VOR BEGINN DER GEISTESKRANKHEIT | | | | |
|---|---|---|---|---|
| Patient Geschlecht | Patient 1 männlich | Patient 2 männlich | Patient 3 weiblich | Patient 4 männlich |
| Alter (Jahre) | 72 | 9 | 22 | 32 |
| Katzen | | | + | + |
| Hunde | + | | + | + |
| Vögel | | + | | |
| and. Tiere | | | + | |

| | | | | |
|---|---|---|---|---|
| ¹⁾ Patient 1 hielt einen Hund 15 Jahre lang vor Ausbruch der manisch-depressiven Krankheit; Patient 2 war in seinem zweiten Lebensjahr, d.h. vor den ersten Anzeichen von Autismus, Vögeln ausgesetzt; Patient 3 und 4 sind mit Hunden und Katzen groß geworden; Patient 3 hatte zusätzlich Hamster und Meerschweinchen. | | | | |

### 2.5 Behandlung von drei Patienten mit unterschiedlichen Grundkrankheiten und einem "begleitenden" depressiven Syndrom

### a) 81-jährige Patientin mit rechtsseitiger Lähmung nach Schlaganfall, der acht Jahre vor Beginn der Zelltherapie stattfand

Zusätzlich zu den Auswirkungen des Schlaganfalls bestand eine Aortenstenose. Die Patientin wurde jede zweite Woche mit ihren eigenen CD3-aktivierten Lymphozyten in einer Dosis von 15 - 20 x 10⁶ Zellen pro Injektion über einen Zeitraum von 12 Monaten behandelt.

Das Ziel der Therapie, eine Verbesserung des depressiven Syndroms, das bei Schlaganfällen häufig zu beobachten ist, aber auch ein Nebenziel, Gehirnzellen wieder zur Signalgebung zu animieren, wurden erreicht. Die Patientin machte auf beidenj Gebieten Fortschritte: der Händedruck der rechten Hand wurde wieder kräftiger, sie konnte den rechten Arm höher heben, sie konnte einige Schritte weiter laufen. Auch die Depression hellte sich auf. Angesichts der unter der Zelltherapie ebenfalls verbesserten Artikulation erfüllte die Patientin sich den Wunsch, italienisch zu lernen. Auch ein Jahr nach Ende der adoptiven Immuntherapie traten bei der Patientin keine Depressionen mehr auf.

Ohne darauf festgelegt werden zu wollen, wird vermutet, daß die bei der Patientin beobachtete Besserung auf die Produktion von Neutrophinen, wie GDNF und BDNF, durch PMBC bewirkt wurde.

### b) 91-jährige Patientin nach Herzinfarkt

Drei Monate nach operativer Behebung einer Aortenstenose bei Vorliegen hoher Antikörpertiter gegen Chlamydien erlitt die Patientin einen kleineren Herzinfarkt und wurde von ihrem Hausarzt digitalisiert. Sie wurde sehr depressiv - eine bekannte Nebenwirkung von Digitalis vor allem bei Patienten über 60 Jahre. Sie konnte erst nach Monate überredet werden, die adoptive Immuntherapie aufzunehmen.

Die Patientin klagte über ein generelles Schwächegefühl und konnte nur langsam einige Schritte gehen. Bereits zwei Wochen nach einer einzigen Behandlung mit aktivierten PBMC war die Patientin fröhlich und lief zum Behandlungszimmer. Der antidepressive Effekt und auch die sehr gute Gehleistung bestehen unvermindert fort.

Die Patientin wird im 2-Wochenrhythmus mit CD3-aktivierten PBMC plus IFNγ und CD3-aktivierten PBMC ohne IFN behandelt, jeweils mit 10-12 x 10⁶ aktivierten Zellen aus einer Zubereitung.

Bezüglich der bei der Patientin beobachteten Besserung wird in Betracht gezogen, daß möglicherweise PBMC auch Katecholamine produzieren, die den Digitaliseffekt auf den Muskel verstärken würden.

### c) 58-jähriger Patient mit multiplen Allergien

Der Patient zeigte nach einem Todesfall in seiner Familie Symptome wie bei einer typischen Depression (Trauerdepression): allgemeines Schwächegefühl, Aktivitätsverlust, u.s.w. In den ersten drei Monaten der Trauer zeigen Hinterbliebene dieselben Symptome wie depressive Patienten, die mit einer schweren Meläncholie in einer psychiatrischen Klinik aufgenommen werden (Paula Clayton, Bereavement and Its Relation to Clinical Depression, in: Results in Depression Research (Hrsg.: Hanns Hippius, Gerry L. Klermann, Norbert Matussek), Springer Verlag, Berlin-Heidelberg 1986). Die Trauerdepression und mit ihr die krankhaften Veränderungen wie die erhöhte Ausschüttung von Kortisol schwächen auch das Immunsystem und erhöhen die Anfälligkeit für verschiedene Krankheiten und Krebs (Paula Clayton, a.a.O.).

Der Patient wurde im ersten Monat einmal wöchentlich mit der unter b) beschriebenen Zellkombination behandelt. Danach wurde die Behandlung 14-tägig über 6 Monate mit einer Zellmenge von 20-25 x 10⁶ Zellen pro Injektion fortgesetzt.

Das Ziel der Therapie, die Minderung der Trauerauswirkungen auf die Handlungs- und Entscheidungsfähigkeit, wurde erreicht. Der Patient beschrieb, daß die schockähnlichen Symptome, wie schwere Glieder und Energielosigkeit, behoben werden konnten, daß aber auch die Zahl der plötzlichen Weinkrämpfe zurückging. Der Zugang zur Trauer wurde jedoch nicht verschüttet, wie das von Antidepressiva mit negativen Spätfolgen bekannt ist.

### 2.6. Behandlung einer Patientin mit Alters-Parkinson-Syndrom

Eine 82-jährige Patientin mit einem Alters-Parkinson-Syndrom litt zusätzlich unter depressiven Verstimmungen, die bei etwa 40% der Patienten auftreten.

Der Patientin wurden in Abständen von zwei Wochen Injektionen von jeweils 15-20 x 10⁶ Zellen, welche mittels anti-CD3-Antikörper stimuliert worden waren, verabreicht. Die Behandlung erfolgte über 5 Jahre.

Bei der Patientin konnten die depressiven Verstimmungen gemildert, aber auch die Feinmotorik verbessert und das Zittern (tremor) wesentlich verringert werden. Bis zum 88. Lebensjahr trat keine Verschlechterung des Zustands der Patientin auf. Die übliche Erhöhung der Parkinsonmedikation (DOPA, Bromocriptin, u.s.w.) mußte nicht vorgenommen werden; im Gegenteil konnte die Medikamentendosis langsam abgebaut werden.

Auf den ersten Blick ist es überraschend, daß verschiedene psychiatrische Erkrankungen oder Störungen, wie manisch-depressive Krankheit, Autismus und Schizophrenie, aber auch Parkinson-Syndrom durch adoptive Immuntherapie auf sehr ähnliche Weise behandelt werden können. Bei näherer Prüfung erkennt man, daß die adoptive Immuntherapie auf die Linderung sehr ähnlicher Symptome abzielt:
- eine Verbesserung der neuromotorischen Fähigkeiten, woran möglicherweise in allen Fällen die gleichen Zytokine beteiligt sein könnten,
- eine Verbesserung der geistigen Beweglichkeit und des Interesses an sozialen Kontakten.

Aus den erzielten Ergebnissen könnte geschlossen werden, daß die stimulierten Immunzellen einfach Zytokine ersetzen, die durch Zellen des ZNS produziert werden sollten, und wenn dem so ist, daß die Zellen des ZNS die Primärziele schädlicher Umwelteinflüsse bei diesen Patienten sein könnten. Es ist unbestritten, daß die Entwicklung von Schizophrenie und depressiver Erkrankungen sowohl genetische als auch Umweltfaktoren erfordert. Die genetischen Faktoren könnten auch die Gene des Immunsystems sein, die die Immunantworten auf Umweltfaktoren steuern. Dieser Ansatz würde Zellen des Immunsystems als Primärziele von Umweltfaktoren postulieren. Zell-Subpoplationen des Immunsystems, die durch einen bestimmten Umweltfaktor inaktiviert würden, könnten nicht länger mit dem ZNS kommunizieren und eine Überproduktion bestimmter Zytokine stimulieren, ähnlich der hormonellen Situation in der Menopause nach Beendigung der Produktion von Hormonen durch die Eierstöcke. Eine derartige regulatorische Überproduktion könnte eine Erklärung für die erhöhten IL-2-Konzentrationen in der Cerebrospinalflüssigkeit schizophrener Patienten sein.

Die Beobachtungen der therapeutischen Wirkungen bei den vier Patienten unterstützen beide Ansätze. Das kontinuierliche Erfordernis für eine adoptive Immuntherapie, um das Ausmaß der Leistungsfähigkeit bei den schizophrenen Patienten 3 und 4 in Form einer Beschäftigung sicherzustellen, legt einen Zytokinersatz durch Immunzellen nahe, obwohl der Ersatz von Zytokinen des Zentralnervensystems durch Immunzellen nicht erklären kann, welches System zuerst versagte.

Die positive therapeutische Wirkung von Azithromycin bei Patient 4 legt eine fundamentale Rolle persistierender Infektionen nahe, die zu einer Polarisierung der Immunantwort und einer Störung des Zytokingleichgewichts führen. Dies wird z.B. durch das Fehlen psychodynamischer Wirkungen von Azithromycin bei Patient 4 unterstützt. Bei Vergleich von Patient 3 und Patient 4 ist festzuhalten, daß der Typ der Chlamydieninfektion sehr wohl die Wirkung auf das geistige Wohlbefinden beeinflussen könnte. Neurologische Folgeerscheinungen von Chlamydieninfektionen sind hauptsächlich den psittaci-Stämmen zugeschrieben worden (Zumula, A., et al., J. Neurol. Neurosurg. Psychiatry 1988: 51, 1462; Williams, W., & Sunderland, R., Archs. Dis. Child. 1989: 64, 1626-1628; Shee, C.D., Postgrad. Med. J. 1988: 64, 382-383). Chlamydia pneumoniae wird neuerdings nicht mehr als psittaci-Stamm klassifiziert.

Ein weiterer interessanter Gesichtspunkt ist, ob zoonotische Infektionen, die bei Haustieren üblich sind, wie Chlamydia psittaci, zu einer langsamen Beeinträchtigung der zellulären Immunantwort führen und aufgrunddessen die Manifestierung psychiatrischer Erkrankungen, wie den vorstehend genannten, unterstützen oder sogar verursachen können. Sämtliche behandelten Patienten hatten zumindest für einen Zeitraum von einem Jahr Kontakt zu Haustieren.

Somit besteht u.a. die Frage, ob die chronisch reaktivierte EBV-Infektion die mentalen Probleme des schizophrenen Patienten 4 verschlimmert. Es ist bekannt, daß exprimiertes BCRF-1-Protein von EBV IL-10 imitiert und die IFNγ-Produktion aktivierter Lymphozyten auf die gleiche Weise wie IL-10 inhibiert (Di-Hwei, H., et al., Science 1990: 259, 830-832). Das BCRF-Polypeptid könnte für die beobachtete Abnahme der IL-2-Produktion von CD3-stimulierten Lymphozyten von schizophrenen Patienten durch Inhibierung des TH-1-Stoffwechselweges über eine Suppression der IFNγ-Produktion verantwortlich sein (Hornberg Merck, et al., Schiz. Res. 1995: 15, 237-242). Dies paßt mit der Beobachtung zusammen, daß anti-CD3-stimulierte Zellen das geistige Wohlbefinden des Patienten 4 nur dann verbesserten, wenn die Zellaktivierung gemäß 1.3, nämlich durch Zusatz von geringen Konzentrationen von IFNγ zu den Zellkulturen zur Unterstützung des TH-1-Stoffwechselweges, ausgeführt worden war. Eine ähnliche Auswirkung auf das geistige Wohlbefinden wurde bei der Zugabe von TFNα zu CD3-stimulierten Zellen festgestellt. Auch eine Behandlung mit beiden Präparationen, d.h. αCD-3 ohne und αCD-3 mit IFNγ bzw. IFNα zur Zellkultur und Injektion an verschiedenen Stellen (rechter und linker Unterarm) scheinen die positive Wirkung zu steigern. Dies könnte eine direkte Wirkung auf die durch EBV-infizierten B-Lymphozyten bedeuten, die Peptide aus der lytischen Phase präsentieren. EBV wird als der hauptsächliche pathogene Faktor bei den meisten Plasmozytomen angesehen und IFNα ist das einzige Interferon, das eine Wirkung auf diesen Tumor hat.

Bei dem Versuch, die Wirkungen eines Virus oder eines anderen pathogenen Faktors auf die Zytokinproduktion und das geistige Wohlbefinden zu bestimmen, sollte berücksichtigt werden, daß die Immunreaktivität hauptsächlich durch drei Faktoren beeinflußt werden kann: 1) durch die immungenetische Konstellation, d.h. durch die Gene des HLA-Komplexes, der für die Prozessierung und Präsentation verantwortlich ist; 2) das Vorhandensein oder Fehlen des schädlichen Umweltfaktors, (bakterielle oder virale Pathogene, Umweltallergene und andere allergisierende Stoffe, ..; dies wird durch die Tatsache belegt, daß nur vierzig Prozent eineiiger Zwillinge das Schicksal teilen, an Schizophrenie zu erkranken; und 3) durch Erwerb einer ungünstigen Kombination chronischer Infektionen, die das Immunsystem polarisieren und die Zytokinkonzentrationen dauerhaft aus dem Gleichgewicht bringen.

Wiederholte Immunisierungen verbesserten die jeweiligen Geisteskrankheit, führten aber auch zum Verschwinden nächtlicher Schweißausbrüche bei den Patienten 1 und 4 und zum Verschwinden häufiger "viraler" Infektionen bei Patient 3 und häufiger Erkältungskrankheiten bei Patient 4. Der Erfolg der adoptiven Immuntherapie zur Beseitigung "immunologischer" Probleme offenbart aber noch immer nicht das Ausmaß von deren Beteiligung an den Geisteskrankheiten. Da mehrere Zytokine, die durch stimulierte Immunzellen produziert werden, identisch mit Nervenwachstumsfaktoren, wie GDNF und anderen, sind, könnte die adoptive Immuntherapie ihre Wirkungen auf dreierlei Weise entfalten: 1) durch Eliminierung von Viren oder Bakterien in infizierten Immunzellen während der in vitro-Kultur; 2) durch Wiederbelebung inaktiverter Lymphozytensubpopulationen und erneute Etablierung eines dynamischen Gleichgewichts zwischen derartigen Subpopulationen ("Depolarisierung polarisierter Subpopulationen), die Zytokine produzieren, die mit dem ZNS kommunizieren; und 3) durch Produktion von Zytokinen, die Nervenzellen schützen.

Der dritte Punkt legt nahe, daß die adoptive Immuntherapie sowohl bei Entwicklungskrankheiten wie auch bei traumatischen Erkrankungen des ZNS nutzbar sein sollte. Sie sollte in nichtimmunologischen Situationen, wie bei Nervenzellschädigungen aufgrund von Unfällen, die zu Koma oder Paraplegie führen, nützlich sein.

Eine Unterentwicklung von ZNS-Strukturen und -Kreisläufen könnte die Folge fehlender Zytokinsignale während der Embryonalentwicklung sein, die auf geringgradigen persistierenden Infektionen beruhen. Die erhöhte Anfälligkeit für Infektionen bei Down-Syndrom hat viel Interesse auf sich gezogen und ist unlängst in einem Übersichtsartikel zusammenfassend dargestellt worden (Nespoli, L., et al., J. Intellect. Disabil. Res. 1993: 37, 543-551). Der positive Einfluß der adoptiven Immuntherapie auf die Entwicklung des autistischen Patienten 2 legt eine positive Wirkung auch bei Down-Syndrom nahe.

## Patentansprüche

1. Verwendung von stimulierten mononukleären Zellen des peripheren Bluts (PBMC) zur Bereitstellung eines Mittels zur Behandlung von mit dem Gehirn assoziierten Erkrankungen, Störungen und Schädigungen, wobei die stimulierten PBMC mittels einer intradermalen oder intramuskulären Injektion verabreicht werden.

2. Verwendung nach Anspruch 1, wobei die mit dem Gehirn assoziierten Erkrankungen, Störungen und Schädigungen eine manisch-depressive Krankheit oder manisch-depressive Psychose, Schizophrenie, depressives Syndrom ohne endogene Ursache, Autismus, Gehirnentwicklungsstörung in und nach der Embryonalzeit, Down-Syndrom oder Parkinson-Syndrom ist oder das Gehirn durch Unfall oder andere Ursachen geschädigt ist.

3. Verwendung nach Anspruch 1 oder 2,
**dadurch gekennzeichnet, dass** die mononukleären Zellen des peripheren Bluts (PBMC) mittels inaktivierter allogener prokaryotischer oder eukaryotischer Zellen stimuliert worden sind.

4. Verwendung nach Anspruch 3,
**dadurch gekennzeichnet, dass** die mononukleären Zellen des peripheren Bluts (PBMC) mittels durch Bestrahlung inaktivierter allogener Zellen stimuliert worden sind.

5. Verwendung nach einem der Ansprüche 1 bis 4,
**dadurch gekennzeichnet, dass** die mononukleären Zellen des peripheren Bluts (PBMC) mittels Proteinen und/oder Peptiden von allogenen prokaryotischen oder eukaryotischen Zellen stimuliert worden sind.

6. Verwendung nach einem der Ansprüche 1 bis 4,
**dadurch gekennzeichnet, dass** die mononukleären Zellen des peripheren Bluts (PBMC) mittels stimulierter T-Lymphozyten stimuliert worden sind.

7. Verwendung nach Anspruch 6,
**dadurch gekennzeichnet, dass** die T-Lymphozyten mittels eines oder mehrerer Antikörper gegen Rezeptoren, Corezeptoren oder Signalmoleküle der T-Lymphozyten stimuliert worden sind.

8. Verwendung nach Anspruch 7,
**dadurch gekennzeichnet, dass** die T-Lymphozyten mittels eines oder mehrerer gegen das CD3-Molekül gerichteter Antikörper stimuliert worden sind.

9. Verwendung nach Anspruch 8,
**dadurch gekennzeichnet, dass** die T-Lymphozyten mittels immobilisierter, gegen das CD3-Molekül gerichteter Antikörper stimuliert worden sind.

10. Verwendung nach Anspruch 6,
**dadurch gekennzeichnet, dass** die T-Lymphozyten mittels eines oder mehrerer Zytokine stimuliert worden sind.

11. Verwendung nach Anspruch 6,
**dadurch gekennzeichnet, dass** die T-Lymphozyten über den αβ-T-Zellrezeptor derselben durch Bindung von durch APC im HLA-Kontext präsentierten Fremdpeptiden stimuliert worden sind.

12. Verwendung nach Anspruch 6,
**dadurch gekennzeichnet, dass** die T-Lymphozyten durch Bindung des B7-1- und/oder des 87-2-Moleküls stimuliert worden sind.

13. Verwendung nach Anspruch 6,
**dadurch gekennzeichnet, dass** die T-Lymphozyten durch Lektine, Calcium-Ionophore und/oder chemischer Analoga derselben und/oder allogene und/oder xenogene Tierzellen stimuliert worden sind.

14. Verwendung nach Anspruch 13,
**dadurch gekennzeichnet, dass** die T-Lymphozyten durch Phytohaem-agglutinin und/oder Concanavalin A stimuliert worden sind.

15. Verwendung nach einem der Ansprüche 6 bis 14,
**dadurch gekennzeichnet, dass** die Stimulierung der T-Lymphozyten in Gegenwart von Interleukin 2 vorgenommen worden ist.

16. Verwendung nach einem der Ansprüche 1 bis 15,
**dadurch gekennzeichnet, dass** nach der Stimulierung der PBMC und/oder T-Lymphozyten eine Behandlung der stimulierten Zellen mit Interferon γ (IFNγ) und/oder Interferon α (IFNα) durchgeführt worden ist.

17. Verwendung nach einem der Ansprüche 1 bis 16,
**dadurch gekennzeichnet, dass** die PBMC durch die folgende Vorgehensweise stimuliert worden sind:
- T-Lymphozyten oder PBMC werden mittels einer oder mehrerer der in den Ansprüchen 3 bis 16 definierten Stimulierungsmethoden primär stimuliert,
- den primär stimulierten T-Lymphozyten oder PBMC werden naive PBMC zugesetzt und zur Stimulierung der naiven PBMC inkubiert.

18. Verwendung nach Anspruch 17,
**dadurch gekennzeichnet, dass** die stimulierten PBMC in einer Dosismenge von 1 - 8 x 10⁶ Zellen, insbesondere 1 - 6 x 10⁶ Zellen pro Injektion eingesetzt werden.

## Claims

1. Use of stimulated peripheral blood mononuclear cells (PBMC) for providing a composition for treating brain-associated diseases, disorders and damages, the stimulated PBMC being administered by means of an intradermal or intramuscular injection.

2. Use according to claim 1 where the brain-associated disease, disorder and damage is a manic-depressive disease or a manic-depressive psychosis, schizophrenia, depressive syndrome without an endogenous cause, autism, a disorder of brain development during and after the prenatal period, Down's syndrome or Parkinson's syndrome, or the brain was damaged by accident or other reasons.

3. Use according to claim 1 or 2, **characterized in that** the peripheral blood mononuclear cells (PBMC) have been stimulated by means of inactivated allogeneic prokaryotic or eukaryotic cells.

4. Use according to claim 3, **characterized in that** the peripheral blood mononuclear cells (PBMC) have been stimulated by means of irradiation of inactivated allogeneic cells.

5. Use according to any of claims 1 to 4, **characterized in that** the peripheral blood mononuclear cells (PBMC) have been stimulated by means of proteins and/or peptides from allogeneic prokaryotic or eukaryotic cells.

6. Use according to any of claims 1 to 4, **characterized in that** the peripheral blood mononuclear cells (PBMC) have been stimulated by means of stimulated T lymphocytes.

7. Use according to claim 6, **characterized in that** the T lymphocytes have been stimulated by means of one or more antibodies against receptors, coreceptors or signal molecules of the T lymphocytes.

8. Use according to claim 7, **characterized in that** the T lymphocytes have been stimulated by means of one or more antibodies directed against the CD3 molecule.

9. Use according to claim 8, **characterized in that** the T lymphocytes have been stimulated by means of immobilized antibodies directed against the CD3 molecule.

10. Use according to claim 6, **characterized in that** the T lymphocytes have been stimulated by means of one or more cytokines.

11. Use according to claim 6, **characterized in that** the T lymphocytes have been stimulated via the αβ-T-cell receptor thereof through binding of foreign peptides presented by APC in the HLA context.

12. Use according to claim 6, **characterized in that** the T lymphocytes have been stimulated by binding of the B7-1 and/or B7-2 molecule.

13. Use according to claim 6, **characterized in that** the T lymphocytes have been stimulated by lectins, calcium ionophores and/or chemical analogues thereof and/or allogeneic and/or xenogeneic animal cells.

14. Use according to claim 13, **characterized in that** the T lymphocytes have been stimulated by phytohemagglutinin and/or concanavalin A.

15. Use according to any of claims 6 to 14, **characterized in that** the stimulation of the T lymphocytes has been carried out in the presence of interleukin 2.

16. Use according to any of claims 1 to 15, **characterized in that** the stimulation of the PBMC and/or T lymphocytes has been followed by a treatment of the stimulated cells with interferon γ (IFNγ) and/or interferon α (IFNα).

17. Use according to any of claims 1 to 16, **characterized in that** the PBMC have been stimulated by the following procedure:
- T lymphocytes or PBMC undergo primary stimulation by means of one or more of the stimulation methods defined in claims 3 to 16;
- naive PBMC are added to the T lymphocytes or PBMC which have undergone primary stimulation, and incubated to stimulate the naive PBMC.

18. Use according to claim 17, **characterized in that** the stimulated PBMC are employed in an amount of 1-8 x 10⁶ cells, in particular 1-6 x 10⁶ cells per injection dose.

## Revendications

1. Utilisation de cellules mononucléaires stimulées du sang périphérique (PBMC) pour le traitement d'affections, de perturbations et de lésions associées au cerveau, les PBMC stimulées ayant été administrées par injection intradermique ou intramusculaire.

2. Utilisation selon la revendication 1, les affections, les perturbations et les lésions associées au cerveau consistant en une maladie ou psychose maniaco-dépressive, une schizophrénie, un syndrome dépressif sans cause endogène, un autisme, des perturbations du développement cérébral pendant et après la période embryonnaire, un syndrome de Down ou un syndrome parkinsonien ou des lésions du cerveau dues à un accident ou à d'autres causes.

3. Utilisation selon la revendication 1 ou 2,
**caractérisée en ce que** les cellules mononucléaires stimulées du sang périphérique (PBMC) ont été stimulées avec des cellules eucaryotes ou procaryotes allogéniques inactivées.

4. Utilisation selon la revendication 3,
**caractérisée en ce que** les cellules mononucléaires stimulées du sang périphérique (PBMC) ont été stimulées avec des cellules allogéniques inactivées par rayonnement.

5. Utilisation selon l'une des revendications 1 à 4,
**caractérisée en ce que** les cellules mononucléaires stimulées du sang périphérique (PBMC) ont été stimulées avec des protéines et/ou des peptides de cellules eucaryotes ou procaryotes allogéniques.

6. Utilisation selon l'une des revendications 1 à 4,
**caractérisée en ce que** les cellules mononucléaires stimulées du sang périphérique (PBMC) ont été stimulées avec des lymphocytes T stimulés.

7. Utilisation selon la revendication 6,
**caractérisée en ce que** les lymphocytes T ont été stimulés avec un ou plusieurs anticorps contre des récepteurs, corécepteurs ou molécules signalétiques des lymphocytes T.

8. Utilisation selon la revendication 7,
**caractérisée en ce que** les lymphocytes T ont été stimulés avec un ou plusieurs anticorps dirigés contre la molécule CD3.

9. Utilisation selon la revendication 8,
**caractérisée en ce que** les lymphocytes T ont été stimulés avec des anticorps immobilisés et dirigés contre la molécule CD3.

10. Utilisation selon la revendication 6,
**caractérisée en ce que** les lymphocytes T ont été stimulés avec une ou plusieurs cytokines.

11. Utilisation selon la revendication 6,
**caractérisée en ce que** les lymphocytes T ont été stimulés via le récepteur αβ des cellules T par liaison de peptides exogènes présentés par les cellules APC dans le contexte HLA.

12. Utilisation selon la revendication 6,
**caractérisée en ce que** les lymphocytes T ont été stimulés par liaison de la molécule B7-1 et/ou B7-2.

13. Utilisation selon la revendication 6,
**caractérisée en ce que** les lymphocytes T ont été stimulés par des lectines, des ionophores de calcium et/ou des analogues chimiques à ces derniers et/ou des cellules animales allogéniques et/ou xénogéniques.

14. Utilisation selon la revendication 13,
**caractérisée en ce que** les lymphocytes T ont été stimulés par phytohémagglutinine et/ou concanavaline A.

15. Utilisation selon l'une des revendications 6 à 14,
**caractérisée en ce que** la stimulation des lymphocytes T a été effectuée en présence d'interleukine 2.

16. Utilisation selon l'une des revendications 1 à 15,
**caractérisée en ce que**, une fois la stimulation des PBMC et/ou des lymphocytes T achevée, les cellules stimulées ont été traitées par interféron γ (IFNγ) et/ou interféron α (IFNα).

17. Utilisation selon l'une des revendications 1 à 16,
**caractérisée en ce que** les PBMC ont été stimulées de la manière suivante :
- Les lymphocytes T ou les PBMC sont tout d'abord stimulés par une ou plusieurs méthodes de stimulation définies dans les revendications 3 à 16,
- Les lymphocytes T ou les PBMC tout d'abord stimulés sont ensuite additionnés de PBMC naïves puis incubés en vue de la stimulation des PBMC naïves.

18. Utilisation selon la revendication 17,
**caractérisée en ce que** la dose des PBMC stimulées administrées est de
1 - 8 x 10⁶ cellules, notamment 1 - 6 x 10⁶ cellules par injection.
